# EUROPEAN PATENT SPECIFICATION

(11) **EP 4 070 721 B1**
(45) Date of publication and mention of the grant of the patent: **24.09.2025**
(21) Application number: 21743973.6
(22) Date of filing: 05.01.2021
(51) Int. Cl.: A61B 5/00, A61B 5/021, A61B 5/022, A44C 5/00

(54) **WEARABLE DEVICE**
AM KÖRPER TRAGBARE VORRICHTUNG
DISPOSITIF POUVANT ÊTRE PORTÉ

(30) Priority: 23.01.2020 CN 202010076893
(43) Date of publication of application: 12.10.2022
(73) Proprietor: Huawei Technologies Co., Ltd., Shenzhen, Guangdong 518129 (CN)
(72) Inventor: HUANG, Zhenlong, Shenzhen, Guangdong 518129 (CN); FU, Xiaoyu, Shenzhen, Guangdong 518129 (CN); WU, Huangwei, Shenzhen, Guangdong 518129 (CN)
(74) Representative: Pfenning, Meinig & Partner mbB
(86) International application number: PCT/CN2021/070331
(87) International publication number: WO 2021/147664

(56) References cited:
- WO-A1-2019/054123
- CN-A- 108 324 261
- CN-A- 109 700 444
- CN-A- 109 745 022
- CN-A- 109 745 023
- CN-A- 110 113 992
- CN-U- 204 394 494
- CN-U- 204 765 615
- CN-U- 206 026 323
- CN-U- 208 081 211
- CN-U- 208 464 067
- CN-U- 208 625 691
- US-A1- 2015 025 400
- US-A1- 2017 049 342
- US-A1- 2019 328 324

## Description

### TECHNICAL FIELD

This application relates to the field of wearable device technologies, and in particular, to a wearable device.

### BACKGROUND

As the pace of people's life accelerates, increasing patients are suffered from abnormal blood pressure, and the abnormal blood pressure easily induces various diseases. Therefore, instant detection of a blood pressure status is essential, and wrist sphygmomanometers emerge. However, in an existing wrist sphygmomanometer, a detection part of the sphygmomanometer is directly connected to an air nozzle of an airbag. In a process of assembling or disassembling a compression band accommodating the airbag, the detection part is easily affected and displaced, which causes damage to the detection part.
US 2019/328324 A1 discloses that a sphygmomanometer includes a cuff and a main body. The main body is equipped with a substrate, a pump and a pressure sensor that are attached to one surface of the substrate, and a plate-shaped member opposed to another surface of the substrate on an opposite side of the one surface of the substrate. The substrate includes a first through-hole and a second through-hole. The other surface of the substrate and an opposing surface of the plate-shaped member opposed to the other surface constitute the planar direction passage extending in a planar direction along the other surface of the substrate. The planar direction passage communicates with a fluid bag of the cuff.
CN 109 700 444 A discloses a blood pressure measuring device and an integrated air pump. The air inlet and outlet passages of a gas charging and discharging device in the blood pressure measuring device are subjected to sealed butt joint with the corresponding air holes on an outer casing or protrude from the corresponding air holes in the outer casing. When the gas charging and discharging device is inflated, the gas outside the outer casing directly enters the inside of the device through the air inlet passage. When deflated, the gas is directly discharged to the outside of the outer casing through the air outlet passage.

### SUMMARY

This application provides a wearable device to avoid a problem that an internal component of the wearable device is damaged due to a displacement of a compression band during assembly or disassembly of the compression band, which prolongs a service life of the wearable device.

The wearable device provided in this application includes a watch face, a watch band, and a compression band. The watch face includes a bezel, a bottom cover, and a blood pressure measurement assembly. The bezel is connected to a peripheral edge of the bottom cover and encloses a watch face inner cavity together with the bottom cover. The bottom cover is provided with an outer plug-connection port, a flow passage, and an inner plug-connection port that successively communicate, and the outer plug-connection port is closer to the bezel than the inner plug-connection port. The blood pressure measurement assembly is accommodated in the watch face inner cavity, and an air nozzle of the blood pressure measurement assembly mutually communicates with the inner plug-connection port. The watch band is connected to the bezel, the compression band and the watch band are stacked, and an air nozzle of the compression band mutually communicates with the outer plug-connection port.

In the wearable device illustrated in this application, the air nozzle of the blood pressure measurement assembly communicates with the inner plug-connection port, the air nozzle of the compression band communicates with the outer plug-connection port, and communication is implemented between the air nozzle of the blood pressure measurement assembly and the air nozzle of the compression band by using the flow passage. That is, the flow passage isolates the air nozzle of the compression band from the air nozzle of the blood pressure measurement assembly, and the blood pressure measurement assembly is not easily affected and displaced in a process of assembling or disassembling the compression band, so as to avoid a problem that the blood pressure measurement assembly is damaged when the compression band is moved, thereby helping prolonging the service life of the wearable device.

In addition, in the wearable device illustrated in this application, the outer plug-connection port, the flow passage, and the inner plug-connection port are all integrated in the bottom cover of the watch face, and there is no need to dispose a communication pipeline in the watch face inner cavity. This reduces space usage of the communication pipeline in the watch face inner cavity, helps increase space utilization of the watch face inner cavity, implements a light and thin design of the watch face, and improves appearance exquisiteness of the wearable device.

In addition, in the wearable device illustrated in this application, the outer plug-connection port is closer to the bezel than the inner plug-connection port, that is, the outer plug-connection port communicating with the air nozzle of the compression band is closer to an edge. The air nozzle of the compression band can communicate with the air nozzle of the blood pressure measurement assembly without extending to directly under the blood pressure measurement assembly. An extension length of the compression band under the watch face can be reduced, and this helps reduce an overall thickness of the wearable device and implement a light and thin design of the wearable device. Moreover, a position between the compression band and the blood pressure measurement assembly can be more flexibly arranged, and components located in the watch face inner cavity such as a battery and a heart rate detection sensor can be more flexibly arranged, so as to help increase the space utilization of the watch face inner cavity and implement a light and thin design of the watch face.

The bottom cover is provided with an inner plug-connection nozzle and an outer plug-connection nozzle, the inner plug-connection port is formed on an inner side of the inner plug-connection nozzle, and the outer plug-connection port is formed on an inner side of the outer plug-connection nozzle. The air nozzle of the blood pressure measurement assembly mutually communicates with the inner plug-connection port, and this means that the air nozzle of the blood pressure measurement assembly and the inner plug-connection nozzle are plug-connected to each other, to be specific, the air nozzle of the blood pressure measurement assembly is plug-connected to the inner plug-connection port, or the air nozzle of the blood pressure measurement assembly is sleeved with the inner plug-connection nozzle. Similarly, the air nozzle of the compression band mutually communicates with the outer plug-connection port, and this means that the air nozzle of the compression band and the outer plug-connection nozzle are plug-connected to each other, to be specific, the air nozzle of the compression band is plug-connected to the outer plug-connection port, or the air nozzle of the compression band is sleeved with the outer plug-connection nozzle.

The compression band includes a cuff, an airbag accommodated in the cuff, and an air nozzle communicating with the airbag.

The airbag and the air nozzle are integrally formed, or the airbag and the air nozzle are formed through assembly.

In an implementation, a cross-sectional area of the flow passage is greater than or equal to 1 mm² to reduce air resistance in the flow passage, ensure an air exchange rate between the air nozzle of the compression band and the air nozzle of the blood pressure detection assembly, implement fast air circulation between the air nozzle of the compression band and the air nozzle of the blood pressure detection assembly, and improve blood pressure detection efficiency of the wearable device.

In an implementation, the watch face further includes a top cover, and the top cover is fixed to a side of the bezel that is away from the bottom cover.

The top cover may be a display. The display includes a display surface that is away from the bezel, and the display surface is configured to display information such as time, an icon, or a physiological parameter of a user.

In an implementation, the bottom cover includes a top face that faces the watch face inner cavity, a distance between the outer plug-connection port and the top face of the bottom cover forms a first distance range, a distance between the inner plug-connection port and the top face of the bottom cover forms a second distance range, and the second distance range partially or totally overlaps the first distance range. In other words, the outer plug-connection port and the inner plug-connection port share a part or all of a thickness distance of the bottom cover in a Z direction, that is, the outer plug-connection port and the inner plug-connection port are relatively close to each other in the Z direction. This helps reduce a distance between the compression band and the blood pressure measurement assembly in the Z direction and reducing a thickness of the bottom cover, thereby helping implement a light and thin design of the wearable device.

A width direction of the watch face is an X direction, a length direction of the watch face is a Y direction, and a thickness direction of the watch face is the Z direction. Every two of the X direction, the Y direction, and the Z direction are perpendicular to each other.

The Z direction is a direction from the top cover to the bottom cover of the watch face.

The top face of the bottom cover is parallel to an X-Y plane of the watch face.

A distance between one end of the outer plug-connection port and the top face of the bottom cover forms one endpoint of the first distance range, and a distance between the other end of the outer plug-connection port and the top face of the bottom cover forms the other endpoint of the first distance range.

A distance between one end of the inner plug-connection port and the top face of the bottom cover forms one endpoint of the second distance range, and a distance between the other end of the inner plug-connection port and the top face of the bottom cover forms the other endpoint of the second distance range.

In an implementation, a third distance range is formed between the flow passage and the top face of the bottom cover, and the third distance range is a combination set of the first distance range and the second distance range, that is, a distance between any position of the flow passage and the top face of the bottom cover always falls within the first distance range or the second distance range. This enables the flow passage to always share a thickness distance of the bottom cover in the Z direction with the outer plug-connection port or the inner plug-connection port, that is, prevents the flow passage from occupying an additional thickness distance of the bottom cover in the Z direction, and helps reduce the thickness of the bottom cover and further helps implement a light and thin design of the watch face.

In an implementation, the air nozzle of the blood pressure measurement assembly extends in the Z direction.

In an implementation, the flow passage is hyphen-shaped, S-shaped, L-shaped, or Z-shaped.

In an implementation, the bottom cover includes a main body member and a first covering member, a peripheral edge of the main body member is connected to the bezel, the first covering member is fixed to one side of the main body member, and the first covering member forms the inner plug-connection port and forms the flow passage through enclosure together with the main body member, so as to simplify a process of forming the flow passage, that is, simplify a process of forming the bottom cover, thereby reducing a preparation cost of the bottom cover.

The main body member is prepared by using plastic such as polycarbonate or a metal material such as stainless steel.

In an implementation, the main body member includes a top face that faces the watch face inner cavity, the top face of the main body member partially protrudes to form a boss, and the first covering member is fixedly connected to the boss and forms the flow passage through enclosure together with the boss.

In the wearable device illustrated in this implementation, the boss formed by partially protruding from the top face of the main body member forms the flow passage together with the first covering member. Only a partial thickness of the bottom cover needs to be increased, instead of increasing an overall thickness of the bottom cover, thereby reducing the preparation cost of the bottom cover. In addition, this design allows the compression band to be connected to the outer plug-connection port at an edge of the watch. This reduces a volume of the compression band stacked at a bottom of the watch, and facilitates a light and thin design of the wearable device.

An elastic modulus of the main body member may be the same as or different from that of the first covering member.

In an implementation, the air nozzle of the blood pressure measurement assembly is plug-connected to the inner plug-connection port, and an elastic modulus of the air nozzle of the blood pressure measurement assembly is greater than an elastic modulus of the first covering member. That is, the air nozzle of the blood pressure measurement assembly is prepared by using a rigid material, and the first covering member is prepared by using an elastic material. To be specific, the air nozzle of the blood pressure measurement assembly is prepared by using a relatively hard material, and the first covering member is prepared by using a relatively soft material. When the air nozzle of the blood pressure measurement assembly is inserted into the inner plug-connection port, the first covering member deforms and closely adheres to the air nozzle of the blood pressure measurement assembly as being squeezed by the air nozzle of the blood pressure measurement assembly, thereby ensuring air-tightness between the air nozzle of the blood pressure measurement assembly and the first covering member.

The first covering member is prepared by using an elastic material such as silica gel or TPU (thermoplastic polyurethanes, thermoplastic polyurethanes).

In an implementation, the bottom cover further includes a second covering member, the second covering member is fixed to a side of the main body member that is away from the first covering member, the second covering member forms the outer plug-connection port, the air nozzle of the compression band is plug-connected to the outer plug-connection port, and an elastic modulus of the air nozzle of the compression band is greater than an elastic modulus of the second covering member. That is, the air nozzle of the compression band is prepared by using a rigid material, and the second covering member is prepared by using an elastic material. To be specific, the air nozzle of the compression band is prepared by using a relatively hard material, and the second covering member is prepared by using a relatively soft material. When the air nozzle of the compression band is inserted into the outer plug-connection port, the second covering member deforms and closely adheres to the air nozzle of the compression band as being squeezed by the air nozzle of the compression band, thereby ensuring air-tightness between the air nozzle of the compression band and the second covering member.

The second covering member is prepared by using an elastic material such as silica gel or TPU.

In an implementation, the main body member includes a bottom face that is away from the watch face inner cavity, the bottom face of the main body member is partially recessed to form an assembly groove, a groove wall of the assembly groove partially protrudes to form an outer plug-connection nozzle, the outer plug-connection port is formed on an inner side of the outer plug-connection nozzle, the air nozzle of the compression band is located in the assembly groove, the outer plug-connection nozzle is plug-connected to the air nozzle of the compression band, that is, the air nozzle of the compression band is sleeved with the outer plug-connection nozzle, to implement a connection between the compression band and the main body member.

In addition, an elastic modulus of the main body member is greater than an elastic modulus of the air nozzle of the compression band. That is, the main body member is prepared by using a rigid material, and the air nozzle of the compression band is prepared by using an elastic material. To be specific, the main body member is prepared by using a relatively hard material, and the air nozzle of the compression band is prepared by using a relatively soft material. When the outer plug-connection nozzle is inserted into the air nozzle of the compression band, because the outer plug-connection nozzle is relatively hard and the air nozzle of the compression band is relatively soft, the air nozzle of the compression band deforms and closely adheres to the outer plug-connection nozzle as being squeezed by the outer plug-connection nozzle, thereby ensuring air-tightness between the outer plug-connection nozzle and the air nozzle of the compression band.

In an implementation, an opening of the assembly groove is located on the bottom face of the main body member, the assembly groove is recessed in a direction from the bottom face to the top face of the main body member and runs through a peripheral face of the main body member, and the air nozzle of the compression band is located in the assembly groove. In this case, the compression band shares a part or all of a thickness distance of the main body member in the Z direction, so as to reduce impact of presence of the compression band on the thickness of the wearable device.

In an implementation, the bottom cover further includes a fixing member, the fixing member is fixed to a side of the compression band that is away from the main body member, and abuts against the compression band. This not only prevents the air nozzle of the compression band from falling off the outer plug-connection nozzle and maintains plug-connection stability between the air nozzle of the compression band and the outer plug-connection nozzle, but also prevents the compression band from falling off the assembly groove and improves connection stability between the compression band and the main body member.

In an implementation, the outer plug-connection nozzle is located between the top face of the main body member and the bottom face of the main body member, that is, the outer plug-connection nozzle shares all of the thickness distance of the main body member in the Z direction, so as to reduce impact of presence of the outer plug-connection nozzle on the thickness of the bottom cover, and help implement a light and thin design of the wearable device.

In an implementation, a peripheral face of the main body member partially protrudes to form an outer plug-connection nozzle, the outer plug-connection port is formed on an inner side of the outer plug-connection nozzle, the air nozzle of the compression band is sleeved with the outer plug-connection nozzle, that is, the outer plug-connection nozzle is plug-connected to the air nozzle of the compression band. In this case, the compression band occupies no bottom face space of the bottom cover, and only shares a part or all of the thickness distance of the main body member in the Z direction, so as to reduce impact of presence of the compression band on the thickness of the wearable device, and facilitate a light and thin design of the wearable device.

In addition, an elastic modulus of the air nozzle of the compression band is less than an elastic modulus of the main body member. That is, the air nozzle of the compression band is prepared by using an elastic material, and the main body member is prepared by using a rigid material. To be specific, the air nozzle of the compression band is prepared by using a relatively soft material, and the main body member is prepared by using a relatively hard material. When the outer plug-connection nozzle is inserted into the air nozzle of the compression band, because the outer plug-connection nozzle is relatively hard and the air nozzle of the compression band is relatively soft, the air nozzle of the compression band deforms and closely adheres to the outer plug-connection nozzle as being squeezed by the outer plug-connection nozzle, thereby ensuring air-tightness between the outer plug-connection nozzle and the air nozzle of the compression band.

In an implementation, the wearable device further includes a air sensor, the air sensor is located in the flow passage and fixed to an inner wall of the flow passage, so as to analyze a air component in an ambient environment of the wearable device when the wearable device performs blood pressure measurement, thereby improving functional diversity of the wearable device and improving use experience of a user.

In an implementation, there are two outer plug-connection ports, two flow passages, and two inner plug-connection ports, the two outer plug-connection ports are a first outer plug-connection port and a second outer plug-connection port, the two flow passages are a first flow passage and a second flow passage, the two inner plug-connection ports are a first inner plug-connection port and a second inner plug-connection port, the first outer plug-connection port, the first flow passage, and the first inner plug-connection port successively communicate, and the second outer plug-connection port, the second flow passage, and the second inner plug-connection port successively communicate;
the blood pressure measurement assembly includes an air pump and a pressure sensor, an air nozzle of the air pump mutually communicates with the first inner plug-connection port, and an air nozzle of the pressure sensor mutually communicates with the second inner plug-connection port; and
the compression band includes two air nozzles, one air nozzle mutually communicates with the first outer plug-connection port, so that the air pump delivers a pumped air into the airbag of the compression band through the first flow passage, or an air in the airbag of the compression band enters the air pump through the first flow passage, and is discharged from the air pump; and the other air nozzle communicates with the second outer plug-connection port, so that an air in the airbag of the compression band is delivered to the pressure sensor through the second flow passage for the pressure sensor to sense a pressure change in the airbag, thereby implementing blood pressure measurement.

In this implementation, the air nozzles of the pressure sensor and the air pump respectively communicate with the air nozzles of the compression band through the two flow passages, instead of sharing one flow passage. This not only can improve flexibility of mounting the blood pressure measurement assembly in the watch face inner cavity, but also can prevent air transmission between the pressure sensor and the airbag of the compression band and air transmission between the air pump and the airbag of the compression band from interfering with each other, thereby helping improve measurement sensitivity and precision of the blood pressure measurement assembly in a blood pressure measurement process.

In an implementation, the bezel or the bottom cover is provided with a breather hole, and the breather hole communicates with the watch face inner cavity and an outside of the watch face, so that the air pump sucks an air from an ambient environment through the breather hole in a blood pressure measurement process, and fills the airbag with the air for blood pressure measurement.

In an implementation, the wearable device further includes a heart rate detection sensor, a middle portion of the bottom cover is provided with a mounting hole spaced from the inner plug-connection port, and the heart rate detection sensor is accommodated in the watch face inner cavity, and directly faces the mounting hole or is at least partially accommodated in the mounting hole, so as to send a sensing signal through the mounting hole to measure a heart rate of a user, thereby improving functional diversity of the wearable device, and improving use experience of the user.

### BRIEF DESCRIPTION OF DRAWINGS

To describe technical solutions in embodiments of this application or in the background more clearly, the following describes the accompanying drawings for describing the embodiments of this application or the background.
FIG. 1 is a schematic structural diagram of a wearable device according to an embodiment of this application;
FIG. 2 is a schematic exploded structural diagram of the electronic device shown in FIG. 1;
FIG. 3 is a schematic structural diagram of a watch face in the wearable device shown in FIG. 2;
FIG. 4 is a schematic exploded structural diagram of the watch face shown in FIG. 3;
FIG. 5 is a schematic structural diagram of a bezel in the watch face shown in FIG. 4;
FIG. 6 is a schematic structural diagram of the bezel shown in FIG. 5 that is sectioned in an A-A direction;
FIG. 7 is a schematic structural diagram of the watch face shown in FIG. 3 that is sectioned in a B-B direction;
FIG. 8 is a schematic structural diagram of a bottom cover in the watch face shown in FIG. 4;
FIG. 9 is a schematic structural diagram of the bottom cover shown in FIG. 8 that is sectioned in a C-C direction;
FIG. 10 is a partial schematic structural diagram of a front view of the structure shown in FIG. 9;
FIG. 11 is a schematic exploded structural diagram of the bottom cover shown in FIG. 8;
FIG. 12 is a schematic structural diagram of a main body member in the bottom cover shown in FIG. 11;
FIG. 13 is a schematic structural diagram of the main body member shown in FIG. 12 that is sectioned in a D-D direction;
FIG. 14 is a schematic structural diagram of the main body member shown in FIG. 12 from another perspective;
FIG. 15 is a schematic structural diagram of a first covering member shown in FIG. 11;
FIG. 16 is a schematic structural diagram of the first covering member shown in FIG. 15 that is sectioned in an E-E direction;
FIG. 17 is a schematic structural diagram of an assembly of a fixing member and the main body member in the bottom cover shown in FIG. 11;
FIG. 18 is a schematic structural diagram of the watch face shown in FIG. 3 that is sectioned in an F-F direction;
FIG. 19 is a schematic structural diagram of a compression band in the wearable device shown in FIG. 2;
FIG. 20 is a schematic structural diagram of an assembly of the compression band and the watch face in the wearable device shown in FIG. 2;
FIG. 21a is a schematic structural diagram of the structure shown in FIG. 20 that is sectioned in a G-G direction;
FIG. 21b is an enlarged schematic structural diagram of region H in the structure shown in FIG. 21a;
FIG. 22 is a schematic structural diagram of a bottom cover that is sectioned in a C-C direction in a second wearable device according to an embodiment of this application;
FIG. 23 is a partial schematic structural diagram of a front view of the structure shown in FIG. 22;
FIG. 24 is a schematic structural diagram of a main body member that is sectioned in a D-D direction in a bottom cover in a third wearable device according to an embodiment of this application;
FIG. 25 is a schematic structural diagram of the bottom cover that is sectioned in a C-C direction in the third wearable device according to an embodiment of this application;
FIG. 26 is a schematic structural diagram of an assembly of the bottom cover and a compression band in the third wearable device according to an embodiment of this application;
FIG. 27 is a schematic structural diagram of the structure shown in FIG. 26 that is sectioned in an I-I direction;
FIG. 28 is a schematic structural diagram of a bottom cover that is sectioned in a C-C direction in a fourth wearable device according to an embodiment of this application;
FIG. 29 is a schematic structural diagram of a compression band in the fourth wearable device according to an embodiment of this application;
FIG. 30 is a schematic structural diagram of an assembly structure of the compression band and the bottom cover in an I-I direction in the fourth wearable device according to an embodiment of this application;
FIG. 31 is a schematic exploded structural diagram of a fifth wearable device according to an embodiment of this application;
FIG. 32 is a schematic exploded structural diagram of a watch face in the wearable device shown in FIG. 31; and
FIG. 33 is a partial schematic structural diagram of an assembly structure schematic diagram of a watch face and a compression band that is sectioned in a G-G direction in a sixth wearable device according to an embodiment of this application.

### DESCRIPTION OF EMBODIMENTS

The following describes the embodiments of this application with reference to the accompanying drawings in the embodiments of this application.

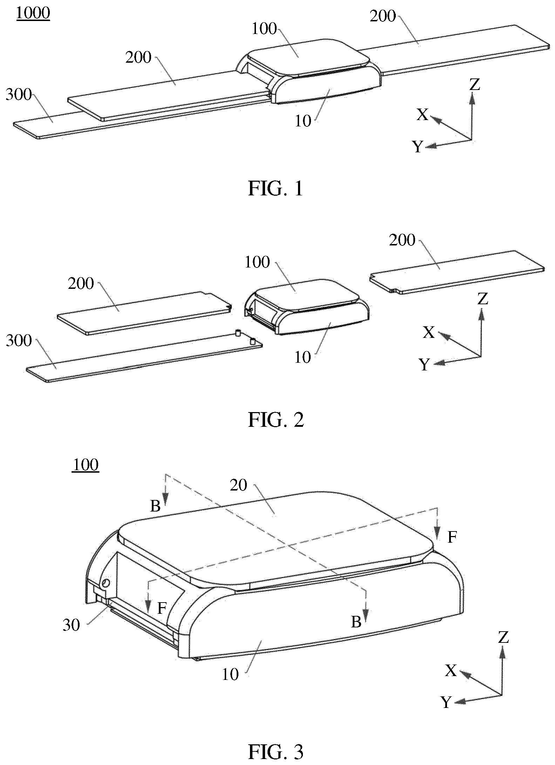

FIG. 1 is a schematic structural diagram of a wearable device 1000 according to an embodiment of this application. FIG. 2 is a schematic exploded structural diagram of the wearable device 1000 shown in FIG. 1.

The wearable device 1000 may be a wrist electronic product such as a watch, a smartwatch, a wristband, or a smart band. The wearable device 1000 in the embodiments shown in FIG. 1 and FIG. 2 is described by using a smartwatch as an example. For ease of description, as shown in FIG. 1, a width direction of the wearable device 1000 is an X direction, a length direction of the wearable device 1000 is a Y direction, and a thickness direction of the wearable device 1000 is a Z direction. Every two of the X direction, the Y direction, and the Z direction are perpendicular to each other.

The wearable device 1000 includes a watch face 100, a watch band 200, and a compression band 300. The watch face 100 includes a bezel 10, and the watch band 200 is connected to the bezel 10. There are two watch bands 200. The two watch bands 200 are a first watch band 200 and a second watch band 200. The first watch band 200 and the second watch band 200 are respectively connected to two opposite sides of the bezel 10. The first watch band 200 is provided with a first locking portion (not shown), and the second watch band 200 is provided with a second locking portion (not shown). The first locking portion and the second locking portion are detachably locked to each other, so that the wearable device 1000 can be worn on a user's wrist. It should be understood that, a mating structure between the first locking portion and the second locking portion may be a watch buckle structure such as a hook buckle, a hidden clasp, a push-button hidden clasp, a leather deployment buckle, a folding clasp with safety, a foldover clasp, or a buckle. This is not specifically limited in this application.

The compression band 300 and the watch band 200 are stacked. Specifically, the compression band 300 is stacked with the first watch band 200 and is located at a bottom of the watch band 200. When the wearable device 1000 is worn by a user, the compression band 300 is located on a side of the watch band 200 and the user's wrist, and adheres to a wrist artery of the user's wrist. It should be noted that, direction terms such as "top" and "bottom" used for the wearable device 1000 illustrated in the embodiments are mainly described based on presentation directions in FIG. 1 and FIG. 2, and do not limit a direction of the wearable device 1000 in an actual application scenario.

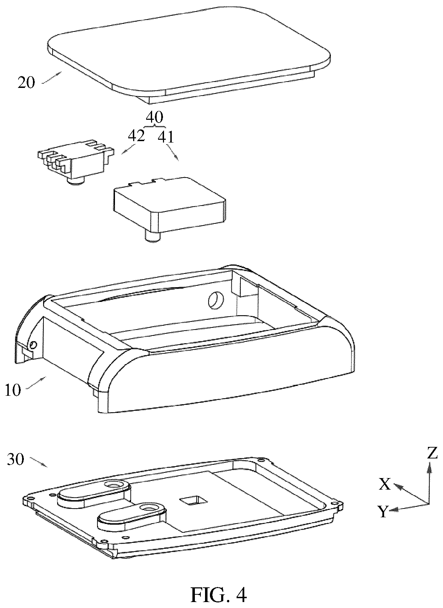

FIG. 3 is a schematic structural diagram of the watch face 100 in the wearable device 1000 shown in FIG. 2. FIG. 4 is a schematic exploded structural diagram of the watch face 100 shown in FIG. 3.

In this embodiment, the watch face 100 is of a cuboid structure. The watch face 100 further includes a top cover 20, a bottom cover 30, a processor (not shown), a blood pressure measurement assembly 40, and a sensor assembly (not shown). The bottom cover 30 and the top cover 20 are located on two opposite sides of the bezel 10. The processor, the blood pressure measurement assembly 40, and the sensor assembly are located between the top cover 20 and the bottom cover 30. The X direction of the wearable device 1000 is a width direction of the watch face 100, the Y direction of the wearable device 1000 is a length direction of the watch face 100, and the Z direction of the wearable device 1000 is a thickness direction of the watch face 100, namely, a direction from the bottom cover 30 to the top cover 20 of the watch face 100. The cuboid structure described above includes a cuboid structure and a structure approximate to a cuboid. The structure approximate to a cuboid means that an outer surface of the cuboid may be partially recessed or partially protruding. The following description of a structure shape may be understood in a same way. It should be understood that, in another embodiment, the watch face 100 may alternatively be cylindrical, conical, cubic, or another geometric shape.

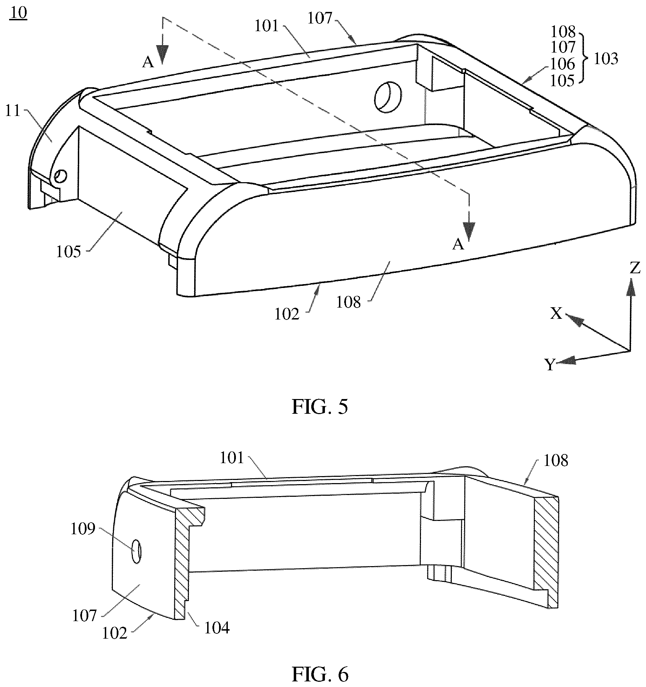

FIG. 5 is a schematic structural diagram of the bezel 10 in the watch face 100 shown in FIG. 4. FIG. 6 is a schematic structural diagram of the bezel 10 shown in FIG. 5 that is sectioned in an A-A direction. It should be noted that, in the accompanying drawings of this application, "sectioned in an A-A direction" means being sectioned along a plane on which line A-A and arrows at both ends of line A-A are located. The following description of the accompanying drawings is understood in a same way.

The bezel 10 is a substantially cuboid frame structure. The bezel 10 includes a top face 101 and a bottom face 102 that are oppositely disposed, and a peripheral face 103 connected between the top face 101 and the bottom face 102. The bottom face 102 of the bezel 10 is partially recessed to form a first fixing groove 104. That is, an opening of the first fixing groove 104 is located on the bottom face 102 of the bezel 10, and the first fixing groove 104 is recessed in a direction from the bottom face 102 to the top face 101 of the bezel 10.

The peripheral face 103 includes a first peripheral face 105 and a second peripheral face 106 that are oppositely disposed, and a third peripheral face 107 and a fourth peripheral face 108 that are connected between the first peripheral face 105 and the second peripheral face 106. The third peripheral face 107 and the fourth peripheral face 108 are oppositely disposed. The first peripheral face 105 partially protrudes to form first fixing lugs 11, and the first fixing lugs 11 protrude from the first peripheral face 105 in a direction away from the second peripheral face 106. There are two first fixing lugs 11, and the two first fixing lugs 11 are spaced at two ends of the first peripheral face 105 in the X-axis direction. In addition, the second peripheral face 106 partially protrudes to form second fixing lugs (not shown), and the second fixing lugs protrude from the second peripheral face 106 in a direction away from the first peripheral face 105. There are two second fixing lugs, and the two second fixing lugs are spaced at two ends of the second peripheral face 106 in the X-axis direction. When the watch bands 200 are connected to the bezel 10, the first watch band 200 is detachably mounted between the two first fixing lugs 11, and the second watch band 200 is detachably mounted between the two second fixing lugs.

The bezel 10 is provided with a breather hole 109. Specifically, the third peripheral face 107 is partially recessed to form the breather hole 109. To be specific, an opening of the breather hole 109 is located on the third peripheral face 107, and the breather hole 109 extends in a direction from the third peripheral face 107 to the fourth peripheral face 108, so that an outside of the bezel 10 communicates with an inside of the bezel 10, to ensure a pressure balance between the inside of the bezel 10 and the outside of the bezel 10. A waterproof breathable film may be disposed in the breather hole 109 to prevent moisture outside the bezel 10 from entering the inside of the bezel 10 through the breather hole 109, and to protect components located inside the bezel 10.

Referring to FIG. 4, the top cover 20 is located on a side of the bezel 10 that is close to the top face 101, and is a substantially rectangular plate structure adapted to the bezel 10. In this embodiment, the top cover 20 is a display. The display includes a display surface 201 that is away from the bezel 10, and the display surface is configured to display information such as time, an icon, or a physiological parameter of a user.

In an implementation, the display includes a cover plate and a display panel fixed to the cover plate. The cover plate may be prepared by using a transparent material such as glass. The display panel may be an LCD (liquid crystal display, liquid crystal display), an OLED (organic light-emitting diode, organic light-emitting diode) display, or an AMOLED (active-matrix organic light-emitting diode, active-matrix organic light-emitting diode or active-matrix organic light-emitting diode) display, a FLED (flex light-emitting diode, flex light-emitting diode) display, a mini LED, a micro LED, a micro OLED, a QLED (quantum dot light emitting diode, quantum dot light emitting diode), or the like. The display panel may further integrate a touch control function. To be specific, the display panel is a touch control display panel, that is, the display panel may be used as not only an input apparatus for receiving input, but also an apparatus for providing output. The display panel is electrically connected to the processor. The display panel can generate a touch control signal and transfer the touch control signal to the processor. The processor receives the touch control signal, and controls enabling of application (application, app) software in the watch face 100 based on the touch control signal. For example, a user may choose to open or edit a graph by touching or pressing a location of the graph on the display.

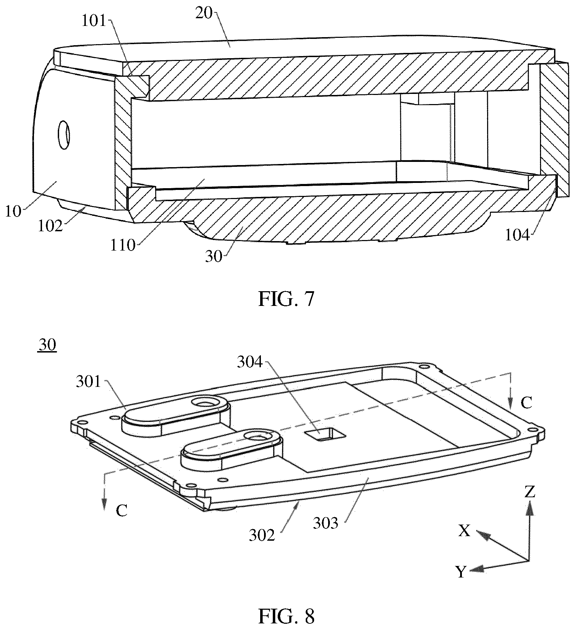

FIG. 7 is a schematic structural diagram of the watch face 100 shown in FIG. 3 that is sectioned in a B-B direction.

The top cover 20 is fixed to the side of the bezel 10 that is close to the top face 101. Specifically, the top cover 20 is fixed to the top face 101 of the bezel 10. An edge of the top cover 20 may be mounted to the top face 101 of the bezel 10 by adhesive. It should be understood that, the top cover 20 is not limited to a 2D (Dimension, dimension) display shown in FIG. 7, and may alternatively be a 2.5D curved screen or a 3D curved screen.

The bottom cover 30 is fixed to a side of the bezel 10 that is away from the top cover 20, that is, the bottom cover 30 is fixed to a side of the bezel 10 that is close to the bottom face 102. Specifically, a peripheral edge of the bottom cover 30 is connected to the bezel 10, and encloses a watch face inner cavity 110 together with the bezel 10. The bottom cover 30 and the top cover 20 are respectively fixed to the two opposite sides of the bezel 10. When the wearable device 1000 is worn by a user, the top cover 20 is disposed away from the user's wrist, and the bottom cover 30 is disposed close to the user's wrist. It should be noted that, the watch face inner cavity 110 is the inside of the bezel 10, and an outside of the watch face 100 is the outside of the bezel 10.

In this embodiment, the peripheral edge of the bottom cover 30 is detachably mounted to the first fixing groove 104, so as to facilitate maintenance and replacement of functional components inside the watch face 100, such as a memory card, a SIM card, and a speaker. In this case, the bezel 10 may be prepared by using a metal alloy material such as titanium alloy or aluminum magnesium alloy. The bottom cover 30 may be prepared by using engineering plastic such as PC (polycarbonate, polycarbonate) or ABS (acrylonitrile butadiene styrene copolymer, acrylonitrile butadiene styrene copolymer), or metal alloy such as titanium alloy or aluminum magnesium alloy. Certainly, the bottom cover 30 and the bezel 10 may be integrally formed, or the bottom cover 30 and the bezel 10 may form an integral structure through assembly, to improve structural stability of the watch face 100. In this case, the bezel 10 and the bottom cover 30 may be prepared by using a metal material.

Refer to FIG. 7 and FIG. 8. FIG. 8 is a schematic structural diagram of the bottom cover 30 in the watch face 100 shown in FIG. 4.

The bottom cover 30 includes a top face 301 that faces the watch face inner cavity 110, a bottom face 302 disposed opposite to the top face 301, and a peripheral face 303 connected between the top face 301 and the bottom face 302. The top face 301 of the bottom cover 30 is parallel to an X-Y plane. It should be understood that, the peripheral edge of the bottom cover 30 refers to an edge portion of the bottom cover 30, that is, a portion in the bottom cover 30 that is formed by an edge of the top face 301 of the bottom cover 30, the peripheral face 303 of the bottom cover 30, and an edge of the bottom face 302 of the bottom cover 30 through enclosure. A peripheral edge of the bottom face 302 is connected to the bezel 10, and this means that the edge of the top face 301 of the bottom cover 30, the peripheral edge of the bottom cover 30, or the edge of the bottom face 302 of the bottom cover 30 is connected to the bezel 10.

A middle portion of the bottom cover 30 is provided with a mounting hole 304. Specifically, the top face 301 of the bottom cover 30 is partially recessed to form the mounting hole 304. To be specific, an opening of the mounting hole 304 is located on the top face 301 of the bottom cover 30, and the mounting hole 304 extends in a direction from the top face 301 to the bottom face 302 of the bottom cover 30 and runs through the bottom face of the bottom cover 30.

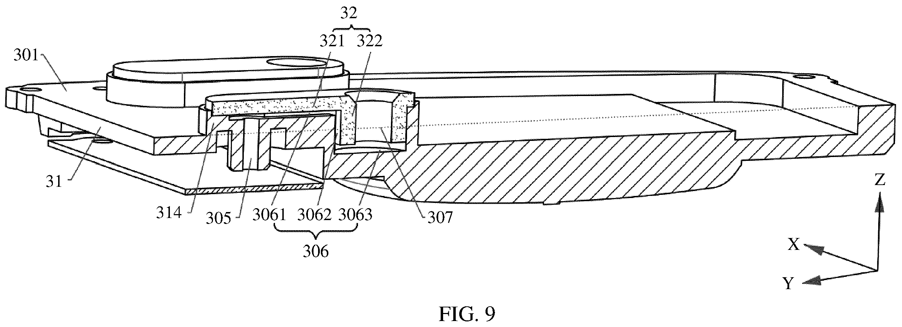

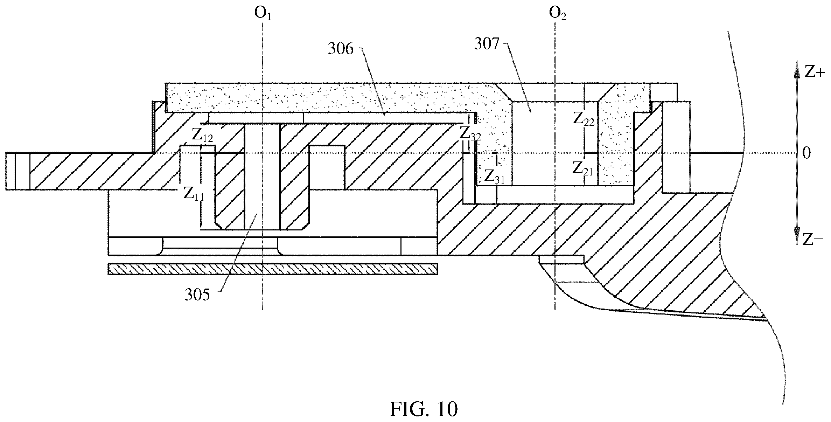

FIG. 9 is a schematic structural diagram of the bottom cover 30 shown in FIG. 8 that is sectioned in a C-C direction. FIG. 10 is a partial schematic structural diagram of a front view of the structure shown in FIG. 9.

The bottom cover 30 is provided with an outer plug-connection port 305, an flow passage 306, and an inner plug-connection port 307 that successively communicate. In this embodiment, there are two outer plug-connection ports 305, two flow passages 306, and two inner plug-connection ports 307. For ease of differentiation, the two outer plug-connection ports 305 are named a first outer plug-connection port 305 and a second outer plug-connection port 305, the two flow passages 306 are named a first flow passage 306 and a second flow passage 306, and the two inner plug-connection ports 307 are named a first inner plug-connection port 307 and a second inner plug-connection port 307. The first outer plug-connection port 305, the first flow passage 306, and the first inner plug-connection port 307 successively communicate, and the second outer plug-connection port 305, the second flow passage 306, and the second inner plug-connection port 307 successively communicate.

For ease of understanding, the following uses the first outer plug-connection port 305, the first flow passage 306, and the first inner plug-connection port 307 as an example for description. In the description below, the outer plug-connection port 305 refers to the first outer plug-connection port 305, the flow passage 306 refers to the first flow passage 306, and the inner plug-connection port 307 refers to the first inner plug-connection port 307. It should be understood that, because structures of the second outer plug-connection port 305 and the first outer plug-connection port 305 are substantially the same, structures of the second flow passage 306 and the first flow passage 306 are substantially the same, and structures of the second inner plug-connection port 307 and the first inner plug-connection port 307 are substantially the same, details are not described repeatedly.

Specifically, the outer plug-connection port 305 is located at an edge position of the bottom cover 30. The outer plug-connection port 305 extends in the Z direction, that is, the outer plug-connection port 305 extends in a thickness direction of the bottom cover 30. That is, an extension direction of the outer plug-connection port 305 is perpendicular to the X-Y plane, in other words, the extension direction of the outer plug-connection port 305 is perpendicular to the top face 301 of the bottom cover 30. The outer plug-connection port 305 has a substantially cylindrical hole structure, and the outer plug-connection port 305 has a central axis O1. It should be understood that, the outer plug-connection port 305 may alternatively have a rectangular hole structure or a hole structure of another shape.

In the following, for ease of understanding a structure of the bottom cover 30, a plane on which the top face 301 of the bottom cover 30 is located is defined as a zero position in the Z direction, an upward direction from the top face 301 of the bottom cover 30 is a Z+ direction, and a downward direction from the top face 301 of the bottom cover 30 is a Z- direction. The top face 301 of the bottom cover 30 is a reference surface for mechanical processing or assembly of the bottom cover 30, that is, the top face 301 of the bottom cover 30 is a positioning reference for mechanical processing or assembly of the bottom cover 30, so as to determine a specific position of each part or component in the watch face 100. It should be noted that, direction terms such as "upper" and "lower" used for the bottom cover 30 illustrated in this embodiment are mainly described based on presentation directions in FIG. 9 and FIG. 10, and do not limit a direction of the bottom cover 30 in an actual application scenario.

A distance between the outer plug-connection port 305 and the top face 301 of the bottom cover 30 forms a first distance range [Z11, Z12]. A distance between one end of the outer plug-connection port 305 and the top face 301 of the bottom cover 30 forms the start point Z11 of the first distance range, and a distance between the other end of the outer plug-connection port 305 and the top face 301 of the bottom cover 30 forms the end point Z12 of the first distance range.

Specifically, the outer plug-connection port 305 includes a lower port and an upper port that are oppositely disposed, and the lower port and the upper port of the outer plug-connection port 305 are respectively located on two sides of the top face 301 of the bottom cover 30. The lower port of the outer plug-connection port 305 is located below the top face 301 of the bottom cover 30, and communicates with an outside of the bottom cover 30. A distance between the lower port of the outer plug-connection port 305 and the top face 301 of the bottom cover 30 is Z11, that is, Z11 is equal to a Z coordinate value of the top face 301 of the bottom cover 30 minus a Z coordinate value of the lower port of the outer plug-connection port 305, where Z11 < 0. The upper port of the outer plug-connection port 305 is located above the top face 301 of the bottom cover 30, and communicates with the flow passage 306. A distance between the upper port of the outer plug-connection port 305 and the top face 301 of the bottom cover 30 is Z12, that is, Z12 is equal to a Z coordinate value of the upper port of the outer plug-connection port 305 minus the Z coordinate value of the top face 301 of the bottom cover 30, where Z12 > 0.

The inner plug-connection port 307 and the outer plug-connection port 305 are spaced. In this embodiment, the inner plug-connection port 307 is located at a middle position of the bottom cover 30, and is spaced from the mounting hole 304. When the bottom cover 30 is connected to the bezel 10, the inner plug-connection port 307 is farther from the bezel 10 than the outer plug-connection port 305, that is, the outer plug-connection port 305 is closer to the bezel 10 than the inner plug-connection port 307.

Specifically, the inner plug-connection port 307 and the outer plug-connection port 305 have a same extension direction, and both extend in the Z direction. That is, the inner plug-connection port 307 also extends in the thickness direction of the bottom cover, in other words, the extension direction of the inner plug-connection port 307 is also perpendicular to the top face 301 of the bottom cover 30, that is, the extension direction of the inner plug-connection port 307 is also perpendicular to the X-Y plane. The inner plug-connection port 307 has a substantially cylindrical hole structure, and the inner plug-connection port 307 has a central axis O2. It should be understood that, the inner plug-connection port 307 may alternatively have a rectangular hole structure or a hole structure of another shape.

A distance between the inner plug-connection port 307 and the top face 301 of the bottom cover 30 forms a second distance range [Z21, Z22]. A distance between one end of the inner plug-connection port 307 and the top face 301 of the bottom cover 30 forms the start point Z21 of the second distance range, and a distance between the other end of the inner plug-connection port 307 and the top face 301 of the bottom cover 30 forms the end point Z22 of the second distance range.

Specifically, the inner plug-connection port 307 includes a lower port and an upper port that are oppositely disposed, and the lower port and the upper port of the inner plug-connection port 307 are respectively located on the two sides of the top face 301 of the bottom cover 30. The lower port of the inner plug-connection port 307 is located below the top face 301 of the bottom cover 30, and communicates with the inside of the bottom cover 30. A distance between the lower port of the inner plug-connection port 307 and the top face 301 of the bottom cover 30 is Z21, that is, Z21 is equal to the Z coordinate value of the top face 301 of the bottom cover 30 minus a Z coordinate value of the lower port of the inner plug-connection port 307, where Z21 < 0. The upper port of the inner plug-connection port 307 is located above the top face 301 of the bottom cover 30, and communicates with the flow passage 306. A distance between the upper port of the inner plug-connection port 307 and the top face 301 of the bottom cover 30 is Z22, that is, Z22 is equal to a Z coordinate value of the upper port of the inner plug-connection port 307 minus the Z coordinate value of the top face 301 of the bottom cover 30, where Z22 > 0.

In this embodiment, the second distance range [Z21, Z22] partially overlaps the first distance range [Z11, Z12]. That is, the outer plug-connection port 305 and the inner plug-connection port 307 are partially stacked in a direction from the central axis O1 of the outer plug-connection port 305 to the central axis O2 of the inner plug-connection port 307. In other words, the outer plug-connection port 305 and the inner plug-connection port 307 share a part of a thickness distance of the bottom cover 30 in the Z direction. In this case, the outer plug-connection port 305 and the inner plug-connection port 307 are relatively close to each other in the Z direction. This can reduce the thickness distance of the bottom cover 30 that is occupied by the outer plug-connection port 305 and the inner plug-connection port 307 in the Z direction, thereby helping reduce a thickness of the bottom cover 30 in the Z direction, and helping implement a light and thin design of the watch face 100.

It should be understood that, in another embodiment, the second distance range [Z21, Z22] may alternatively totally overlap the first distance range [Z11, Z12]. In this case, the lower port of the outer plug-connection port 305 may be flush with the lower port of the inner plug-connection port 307, and the outer plug-connection port 305 and the inner plug-connection port 307 are fully stacked in the direction from the central axis O1 of the outer plug-connection port 305 to the central axis O2 of the inner plug-connection port 307. In other words, the outer plug-connection port 305 and the inner plug-connection port 307 share all of the thickness distance of the bottom cover 30 in the Z direction, that is, the outer plug-connection port 305 is entirely flush with the inner plug-connection port 307 in the Z direction. This minimizes the thickness distance of the bottom cover 30 that is occupied by the outer plug-connection port 305 and the inner plug-connection port 307 in the Z direction, thereby helping implement a light and thin design of the watch face 100.

The flow passage 306 communicates between the outer plug-connection port 305 and the plug-connection port 307. Herein, the flow passage 306 is Z-shaped. It should be understood that, in another embodiment, the flow passage 306 may be hyphen-shaped, S-shaped, L-shaped, or the like.

A third distance range [Z31, Z32] is formed between the flow passage 306 and the top face 301 of the bottom cover 30. Specifically, the flow passage 306 includes a lower end face and an upper end face that are oppositely disposed, and the lower end face and the upper end face of the flow passage 306 are respectively located on the two sides of the top face 301 of the bottom cover 30. The lower end face of the flow passage 306 is located below the top face 301 of the bottom cover 30. A distance between the lower end face of the flow passage 306 and the top face 301 of the bottom cover 30 is Z31, that is, Z31 is equal to the Z coordinate value of the top face 301 of the bottom cover 30 minus a Z coordinate value of the lower end face of the flow passage 306, where Z31 < 0. The upper end face of the flow passage 306 is located above the top face 301 of the bottom cover 30. A distance between the upper end face of the flow passage 306 and the top face 301 of the bottom cover 30 is Z32, that is, Z32 is equal to a Z coordinate value of the upper end face of the flow passage 306 minus the Z coordinate value of the top face 301 of the bottom cover 30, where Z32 > 0.

In this embodiment, the third distance range [Z31, Z32] is a combination set of the second distance range [Z21, Z22] and the first distance range [Z11, Z12], that is, a distance between any position of the flow passage 306 and the top face 301 of the bottom cover 30 always falls within the first distance range [Z11, Z12] or the second distance range [Z21, Z22]. That is, one part of the flow passage 306 is stacked with the inner plug-connection port 307 and the other part of the flow passage 306 is stacked with the outer plug-connection port 305 in the direction from the central axis O1 of the outer plug-connection port 305 to the central axis O2 of the inner plug-connection port 307. In other words, the flow passage 306 share all of the thickness distance of the bottom cover 30 in the Z direction with the inner plug-connection port 307 and the outer plug-connection port 305. This prevents occupation of an additional thickness distance of the bottom cover 30 in the Z direction that is caused by presence of the flow passage 306, and helps reduce the thickness of the bottom cover 30 in the Z direction and implement a light and thin design of the watch face 100.

In an implementation, the flow passage 306 includes a first passage 3061, a second passage 3062b, and a third passage 3063 that successively communicate. The first passage 3061 extends along the X-Y plane, and communicates with the upper port of the outer plug-connection port 305. The second passage 3062b extends in the Z direction, and communicates between the first passage 3061 and the third passage 3063. The third passage 3063 extends along the X-Y plane, and communicates with the lower port of the inner plug-connection port 307. Specifically, a cross-sectional area of the flow passage 306 is greater than 1 mm² to reduce air resistance generated when air flows inside the flow passage 306, thereby ensuring a air flow rate between the outer plug-connection port 305 and the inner plug-connection port 307. A shape of a cross section of the flow passage 306 includes, but is not limited to, a circle, a square, or another geometric shape.

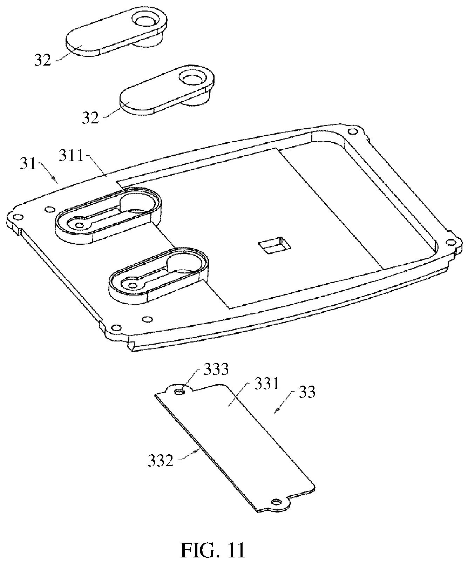

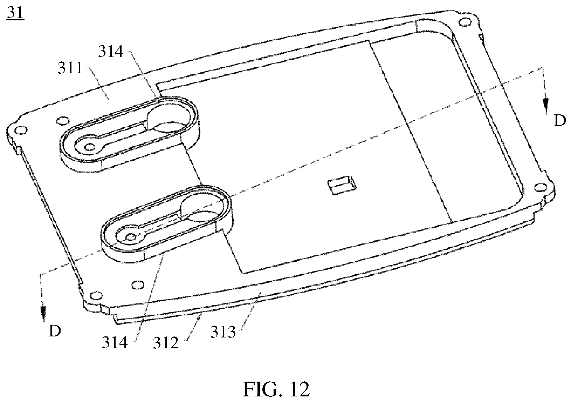

FIG. 11 is a schematic exploded structural diagram of the bottom cover 30 shown in FIG. 8. FIG. 12 is a schematic structural diagram of a main body member 31 in the bottom cover 30 shown in FIG. 11.

In this embodiment, the bottom cover 30 includes the main body member 31, a first covering member 32, and a fixing member 33. The first covering member 32 and the fixing member 33 are located on two opposite sides of the main body member 31. The main body member 31 includes a top face 311 and a bottom face 312 that are oppositely disposed, and a peripheral face 313 connected between the top face 311 and the bottom face 312. The main body member is prepared by using plastic such as polycarbonate (PC, polycarbonate) or a metal material such as stainless steel. It should be understood that, the top face 311 of the main body member 31 is the top face 301 of the bottom cover 30 described above, and the peripheral face 313 of the main body member 31 is the peripheral face 303 of the bottom cover 30 described above.

The top face 311 of the main body member 31 partially protrudes to form a boss 314. In this embodiment, there are two bosses 314, and the two bosses 314 are spaced. For ease of differentiation, the two bosses 314 are named a first boss 314 and a second boss 314. The following describes in detail a structure of the main body member 31 by using the first boss 314 as an example. It should be understood that, the boss 314 mentioned below refers to the first boss 314. Because structures of the second boss 314 and the first boss 314 are substantially the same, details are not described repeatedly below.

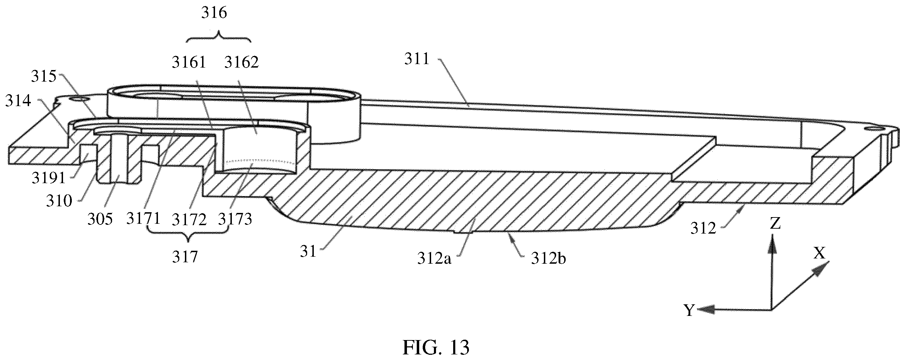

FIG. 13 is a schematic structural diagram of the main body member 31 shown in FIG. 12 that is sectioned in a D-D direction.

The boss 314 includes a top face 315 that has a same orientation as the top face 311 of the main body member 31, and the top face 315 of the boss 314 is partially recessed to form a first mounting groove 316 and a flow groove 317. Specifically, an opening of the first mounting groove 316 is located at a middle position of the top face 315 of the boss 314. The first mounting groove 316 is recessed in a direction from the top face 315 of the boss 314 to the top face 315 of the main body member 31. The first mounting groove 316 is substantially L-shaped. The first mounting groove 316 includes a first groove body 3161 and a second groove body 3162. The first groove body 3161 extends along the X-Y plane. A bottom groove wall of the first groove body 3161 is partially recessed to form the second groove body 3162, that is, an opening of the second groove body 3162 is located on the bottom groove wall of the first groove body 3161. The second groove body 3162 extends in a direction from the bottom groove wall of the first groove body 3161 to the bottom face 312 of the main body member 31, that is, the second groove body 3162 extends in the Z direction.

A groove wall of the first mounting groove 316 is partially recessed to form the flow groove 317, that is, an opening of the flow groove 317 is located on the groove wall of the first mounting groove 316. Specifically, the flow groove 317 is Z-shaped. The flow groove 317 includes a first portion 3171, a second portion 3172, and a third portion 3173 that successively communicate. Openings of both the first portion 3171 and the second portion 3172 are located on the bottom groove wall of the first groove body 3161. Both the first portion 3171 and the second portion 3172 are recessed in a direction from the bottom groove wall of the first groove body 3161 to the bottom face 312 of the main body member 31. The first portion 3171 extends along the X-Y plane, and the second portion 3172 extends in the Z direction and runs through a groove sidewall of the second groove 3162. A bottom groove wall of the second groove body 3162 is recessed in the Z direction to form the third portion 3173, that is, the third portion 3172 is located under the second groove body 3162. The third portion 3172 extends along the X-Y plane.

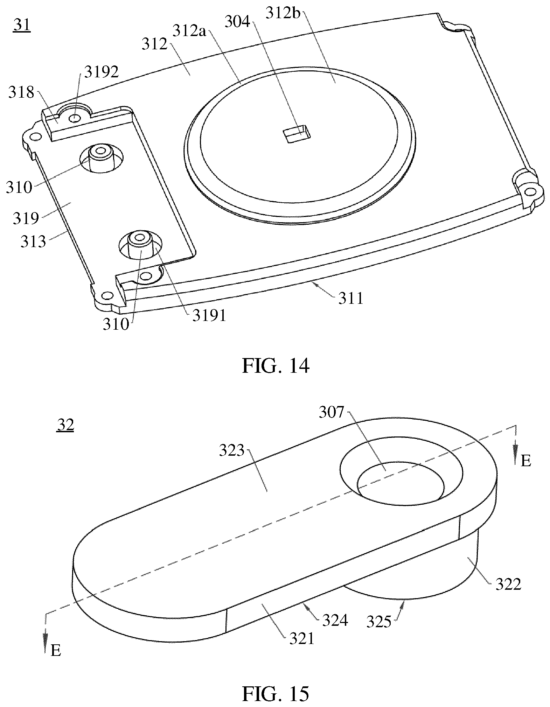

Refer to FIG. 13 and FIG. 14. FIG. 14 is a schematic structural diagram of the main body member 31 shown in FIG. 12 from another perspective.

The bottom face 312 of the main body member 31 partially protrudes to form a round boss 312a. Specifically, the round boss 312a is connected to a middle region of the bottom face 312 of the main body member 31. The round boss 312a protrudes from the bottom face 312 of the main body member 31 in a direction away from the top face 311. The round boss 312a includes a bottom face 312b that has a same orientation as the bottom face 312 of the main body member 31. The mounting hole 309 runs through the bottom face 312b of the round boss 312a. When the wearable device 1000 is worn by a user, the bottom face 312b of the round boss 312a may adhere to the user's wrist.

The bottom face 312 of the main body member 31 is partially recessed to form a second fixing groove 318 and an assembly groove 319. Specifically, an opening of the second fixing groove 318 is located in an edge region of the bottom face 312 of the main body member 31. The second fixing groove 318 is recessed in a direction from the bottom face 312 to the top face 311 of the main body member 31, and runs through the peripheral face 313 of the main body member 31. A bottom groove wall of the second fixing groove 318 is partially recessed to form the assembly groove 319. The assembly groove 319 is recessed in a direction from the bottom groove wall of the second fixing groove 318 to the top face 311 of the main body member 31, and runs through the peripheral face 313 of the main body member 31.

A groove wall of the assembly groove 319 partially protrudes to form an outer plug-connection nozzle 310, the outer plug-connection port 305 is formed on an inner side of the outer plug-connection nozzle 310, and the outer plug-connection port 305 communicates with the first portion 3171 of the flow groove 317. In this embodiment, the outer plug-connection nozzle 310 is provided on a bottom groove wall of the assembly groove 319, that is, the bottom groove wall of the assembly groove 319 partially protrudes to form the outer plug-connection nozzle 310. Certainly, in another embodiment, the outer plug-connection nozzle 310 may alternatively be disposed on a groove sidewall of the assembly groove 319, that is, the groove sidewall of the assembly groove 319 partially protrudes to form the outer plug-connection nozzle 310. It should be understood that, a shape of the outer plug-connection nozzle 310 is not limited to a circular tubular structure shown in FIG. 14, and may alternatively be a square tubular structure or a tubular structure of another geometric shape.

In this embodiment, there are two outer plug-connection nozzles 310. The two outer plug-connection nozzles 310 are a first outer plug-connection nozzle 310 and a second outer plug-connection nozzle 310, and the first outer plug-connection nozzle 310 and the second outer plug-connection nozzle 310 are spaced. The first outer plug-connection port 305 is formed on an inner side of the first outer plug-connection nozzle 310, and the first outer plug-connection port 305 communicates with the first portion 3171 of the flow groove 317 in the first boss 314. The second outer plug-connection port 305 is formed on an inner side of the second outer plug-connection nozzle 310, and the second outer plug-connection port 305 communicates with the first portion 3171 of the flow groove 317 in the second boss 314. For ease of understanding, the following uses the first outer plug-connection nozzle 310 as an example for description. Unless otherwise specified, the outer plug-connection nozzle 310 mentioned below refers to the first outer plug-connection nozzle 310. It should be noted that, structures of the second outer plug-connection nozzle 310 and the first outer plug-connection nozzle 310 are basically the same, and details are not described repeatedly below.

Specifically, the bottom groove wall of the assembly groove 319 is partially recessed to form an avoidance groove 3191. That is, an opening of the avoidance groove 3191 is located on the bottom groove wall of the assembly groove 319. It can be understood that, a shape of the avoidance groove 3191 is not limited to a circular hole structure shown in FIG. 14, and may alternatively be a rectangular or strip-shaped hole structure.

The avoidance groove 3191 is recessed in a direction from the bottom groove wall of the assembly groove 319 to the top face 311 of the main body member 31. A bottom groove wall of the avoidance groove 3191 partially protrudes to form the outer plug-connection nozzle 310, that is, the outer plug-connection nozzle 310 extends in a direction from the bottom groove wall of the avoidance groove 3191 to the bottom groove wall of the assembly groove 319. Specifically, the outer plug-connection nozzle 310 is located between the top face 311 and the bottom face 312 of the main body member 31, that is, the outer plug-connection nozzle 310 shares all of the thickness distance of the main body member 31 in the Z direction, so as to reduce impact of presence of the outer plug-connection nozzle 310 on the thickness of the bottom cover 30, and help implement a light and thin design of the watch face 100.

In this embodiment, because the second distance range [Z21, Z22] only partially overlaps the first distance range [Z11, Z12], the lower port of the outer plug-connection port 305 is not flush with the lower port of the inner plug-connection port 307. In this case, the outer plug-connection nozzle 310 protrudes from the bottom groove wall of the assembly groove 319, but does not exceed the bottom face 312 of the main body member 31, and does not exceed the bottom face 312b of the round boss 312a. Compared with the embodiment in which the second distance range [Z21, Z22] totally overlaps the first distance range [Z11, Z12], in the wearable device 1000 illustrated in this embodiment, in the Z direction, a length size of the outer plug-connection nozzle 310 is larger, and an area of an outer peripheral face of the outer plug-connection nozzle 310 is also larger.

In addition, the bottom groove wall of the assembly groove 319 is partially recessed to form a first fixing hole 3192. Specifically, an opening of the first fixing hole 3192 is located in an edge region of the bottom groove wall of the assembly groove 319, and is spaced from the opening of the avoidance groove 3191. The first fixing hole 3192 is recessed in a direction from the bottom groove wall of the assembly groove 319 to the top face 311 of the main body member 31. There are two first fixing holes 3192, and the two first fixing holes 3192 are spaced and are located on two opposite sides of the avoidance groove 3191.

Referring to FIG. 11, the first covering member 32 is located on a side of the main body member 31 that is close to the top face 311, and the first covering member 32 forms the inner plug-connection port 307. In this embodiment, there are two first covering members 32, and the two first covering members 32 are located on a same side of the main body member 31. Specifically, one first covering member 32 forms the first inner plug-connection port 307, and is adapted to the first boss 314. The other first covering member 32 forms the second inner plug-connection port 307, and is adapted to the second boss 314. Structures of the two first covering members 32 are basically the same.

FIG. 15 is a schematic structural diagram of the first covering member 32 shown in FIG. 11.

The first covering member 32 includes a first covering portion 321 and a first plug-connection portion 322 connected to the first covering portion 321. In this embodiment, the first covering portion 321 has a substantially flat plate structure. The first covering portion 321 includes a top face 323 and a bottom face 324 that are oppositely disposed. The first plug-connection portion 322 is connected to the bottom face 324 of the first covering portion 321. The first plug-connection portion 322 extends from the bottom face 324 of the first covering portion 321 in a direction away from the top face 323, and has a substantially circular tubular structure. The first plug-connection portion 322 includes a bottom face 325 that has a same orientation as the bottom face 324 of the first covering portion 321. The bottom face 325 of the first plug-connection portion 322 is partially recessed to form the inner plug-connection port 307, that is, an opening of the inner plug-connection port 307 is located on the bottom face 325 of the first plug-connection portion 322. The inner plug-connection port 307 extends in a direction from the bottom face 325 of the first plug-connection portion 322 to the top face 323 of the first covering portion 321, and runs through the top face 323 of the first covering portion 321. The first covering member 32 is prepared by using an elastic material such as silica gel or TPU.

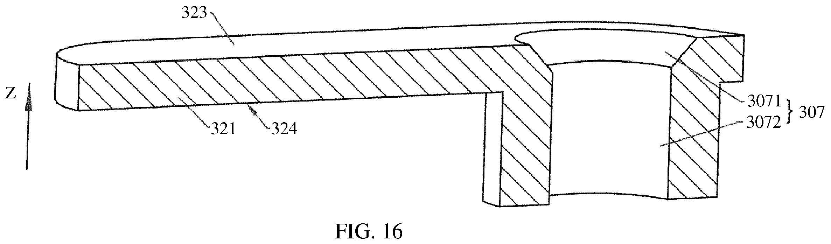

FIG. 16 is a schematic structural diagram of the first covering member 32 shown in FIG. 15 that is sectioned in an E-E direction.

The inner plug-connection port 307 includes an upper end portion 3071 and a lower end portion 3072 that communicate with each other. The upper end portion 3071 is close to the top face 323 of the first covering portion 321. An inner diameter of the upper end portion 3071 gradually increases in a direction from the bottom face 324 to the top face 323 of the first covering portion 321, that is, in the Z direction shown in the figure, which is also a thickness direction of the first covering portion 321. In other words, the upper end portion 3071 has a substantially horn-shaped hole structure. The lower end portion 3072 is located under the upper end portion 3071, and the lower end portion 3072 has a substantially cylindrical hole structure.

Referring to FIG. 9, the first covering member 32 is fixed to one side of the main body member 31, and forms the flow passage 306 together with the main body member 31 through enclosure, so as to simplify a process of forming the flow passage 306, that is, simplify a process of forming the bottom cover 30, thereby reducing a preparation cost of the bottom cover 30. A material of the first covering member 32 is different from a material of the main body member 31, that is, an elastic modulus of the first covering member 32 is different from that of the main body member 31. It should be understood that, the material of the first covering member 32 may alternatively be the same as the material of the main body member 31, that is, the elastic modulus of the first covering member 32 may be the same as the elastic modulus of the main body member 31. In this case, the first covering member 32 may also be integrally formed with the main body member 31, to improve structural strength of the bottom cover 30 and ensure structural stability of the bottom cover 30.

In this embodiment, the first covering member 32 is fixedly connected to the boss 314 and forms the flow passage 306 together with the boss 314. The first covering member 32 with the first inner plug-connection port 307 formed is fixedly connected to the first boss 314, and forms the first flow passage 306 together with the first boss 314. The first covering member 32 with the second inner plug-connection port 307 formed is fixedly connected to the second boss 314, and forms the second flow passage 306 together with the second boss 314.

In the wearable device 1000 illustrated in this implementation, the boss 314 formed by partially protruding from the top face 323 of the main body member 31 forms the flow passage 306 together with the first covering member 32. Only a partial thickness of the bottom cover 30 needs to be increased, instead of increasing an overall thickness of the bottom cover 30, thereby reducing the preparation cost of the bottom cover 30.

The following describes an assembly structure between the first covering member 32 and the boss 314 by using the first covering member 32 with the first inner plug-connection port 307 formed and the first boss 314 as an example. It should be understood that, an assembly structure between the first covering member 32 with the second inner plug-connection port 307 formed and the second boss 314 is substantially the same as an assembly structure between the first covering member 32 with the first inner plug-connection port 307 formed and the first boss 314. Details are not described repeatedly below.

Referring to FIG. 13, the first covering member 32 is fixedly mounted to the first mounting groove 316. The first covering portion 321 is fixedly mounted to the first groove body 3161 of the first mounting groove 316. The first covering portion 321 covers the openings of the first portion 3171 and the second portion 3172 of the flow groove 317 to form the first passage 3061 of the flow passage 306. The first plug-connection portion 322 extends from the first groove body 3161 into the second groove body 3162, so that the inner plug-connection port 307 communicates with the third portion 3173 of the flow groove 317. The first plug-connection portion 322 adheres to the groove sidewall of the second groove body 3162, and covers the opening that is of the second portion 3172 of the flow groove 317 and that is located on the groove sidewall of the second groove body 3162, to form the second passage 3062 of the flow passage 306. The first plug-connection portion 322 also covers an opening of the third portion 3173 of the flow groove 317, to form the third passage 3063 of the flow passage 306.

Referring to FIG. 11, the fixing member 33 is located on a side of the main body member 31 that is away from the first covering member 32, that is, the fixing member 33 is located on a side of the main body member 31 that is close to the bottom face 312. The fixing member 33 has a plate structure adapted to a shape of the assembly groove 319. The fixing member 33 includes a top face 331 and a bottom face 332 that are oppositely disposed. The bottom face 332 of the fixing member 33 is partially recessed to form a second fixing hole 333, that is, an opening of the second fixing hole 333 is located on the bottom face 332 of the fixing member 33. The second fixing hole 333 extends in a direction from the bottom face 332 to the top face 331 of the fixing member 33, and runs through the top face 331 of the fixing member 33, that is, the second fixing hole 333 runs through the fixing member 33 in a thickness direction of the fixing member 33. There are two second fixing holes 333, and the two second fixing holes 333 are spaced on an edge of the fixing member 33.

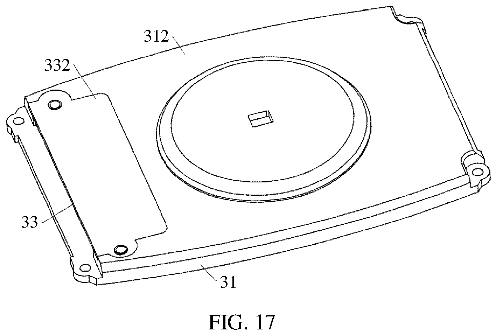

Refer to FIG. 14 and FIG. 17. FIG. 17 is a schematic structural diagram of an assembly of the fixing member 33 and the main body member 31 in the bottom cover 30 shown in FIG. 11.

The fixing member 33 is fixed to the side of the main body member 31 that is away from the first covering member 32. That is, the first covering member 32 and the fixing member 33 are respectively fixed to the two opposite sides of the main body member 31. When the wearable device 1000 is worn by a user, the fixing member 33 is placed and faces the user's wrist, and the first covering member 32 is placed away from the user's wrist.

Specifically, the fixing member 33 is fixedly mounted to the assembly groove 319. The fixing member 33 may use a fastener such as a screw or a bolt to pass through the second fixing hole 333 and be locked to the first fixing hole 3192, so as to implement detachable mounting of the fixing member 33. In this case, the fixing member 33 covers an opening of the assembly groove 319, and covers the avoidance groove 3191 and the outer plug-connection nozzle 310. In addition, the bottom face 332 of the fixing member 33 is flush with the bottom face 332 of the main body member 31, that is, the bottom face 332 of the fixing member 33 and the bottom face 332 of the main body member 31 are located on a same plane, so that when the wearable device 1000 is worn by a user, a joint between the bottom face 332 of the fixing member 33 and the bottom face 332 of the main body member 31 does not protrude partially and abut against the user's wrist, thereby improving use experience of the user.

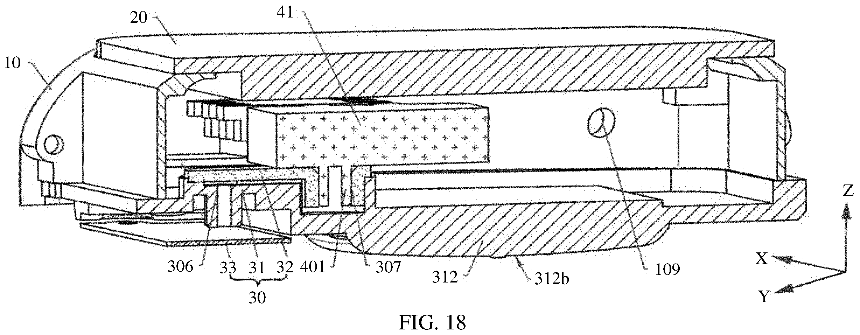

Refer to FIG. 4 and FIG. 18. FIG. 18 is a schematic structural diagram of the watch face 100 shown in FIG. 3 that is sectioned in an F-F direction.

A peripheral edge of the main body member 31 is connected to the bezel 10, and encloses the watch face inner cavity 110 together with the bezel 10 and the top cover 20. In this case, the top face 311 of the main body member 31 faces the watch face inner cavity 110, that is, the boss 314 is located in the watch face inner cavity 110. The first covering member 32 is located in the watch face inner cavity 110, and the fixing member 33 is located outside the watch face 100. Both the first covering member 32 and the boss 314 are located in the watch face inner cavity 110. This fully utilizes a volume of the watch face inner cavity 110 to form the flow passage 306, without adding an additional volume outside the watch face 100, thereby facilitating a light and thin design of the watch face 100.

The processor is accommodated in the watch face inner cavity 110. The processor may include one or more processing units. For example, the processor may include an application processor (Application Processor, AP), a modem processor, a graphics processing unit (Graphics Processing Unit, GPU), an image signal processor (Image Signal Processor, ISP), a controller, a memory, a video codec, a digital signal processor (Digital Signal Processor, DSP), a baseband processor, a neural-network processing unit (Neural-network Processing Unit, NPU), and/or the like. Different processing units may be independent components, or may be integrated into one or more processors. The processor may be a nerve center and a command center of the wearable device 1000. The processor may generate an operation control signal based on an instruction operation code and a time sequence signal to complete control of instruction fetching and instruction execution. In addition, the processor may further include a storage unit, configured to store instructions and data. For example, the storage unit of the processor is a cache. The memory may store an instruction or data just used or cyclically used by the processor. If the processor needs to use the instruction or the data again, the processor may directly invoke the instruction or the data from the memory. This avoids repeated access and reduces waiting time of the processor, thereby improving system efficiency.

The blood pressure measurement assembly 40 is electrically connected to the processor. In this embodiment, the blood pressure measurement assembly 40 includes an air pump 41 and a pressure sensor 42. The air pump 41 is electrically connected to the processor, and is configured to receive a control signal sent by the processor and suck or discharge a air based on the control signal. The pressure sensor 42 is spaced from the air pump 41, and is electrically connected to the processor. The pressure sensor 42 is configured to receive a control signal sent by the processor, sense a pressure signal based on the control signal, and convert the pressure signal into an electrical signal. The pressure sensor may be a capacitive pressure sensor. The capacitive pressure sensor includes at least two parallel plates made of conductive materials. When a force is applied to the pressure sensor, capacitance between electrodes changes. The processor determines pressure intensity based on a change of the capacitance between the electrodes. Certainly, the pressure sensor 42 may alternatively be a resistive pressure sensor, an inductive pressure sensor, or the like.

Specifically, the blood pressure measurement assembly 40 is located in the watch face inner cavity 110, and an air nozzle 401 of the blood pressure measurement assembly 40 mutually communicates with the inner plug-connection port 307. An air nozzle 401 of the air pump 41 mutually communicates with the first inner plug-connection port 307, so that the air pump 41 delivers a pumped air to the first flow passage 306 through the breather hole 109, or receives a air delivered from the first flow passage 306 and discharges the air through the breather hole 109. An air nozzle 401 of the pressure sensor 42 mutually communicates with the second inner plug-connection port 307, so that the pressure sensor 42 senses a pressure change through the second flow passage 306 and performs blood pressure measurement.

The following describes an assembly structure between the air nozzle 401 of the blood pressure measurement assembly 40 and the inner plug-connection port 307 by using the air nozzle 401 of the air pump 41 as an example. The air nozzle 401 of the blood pressure measurement assembly 40 mentioned below refers to the air nozzle 401 of the air pump 41, and the inner plug-connection port 307 mentioned below refers to the first inner plug-connection port 307 that mutually communicates with the air nozzle 401 of the air pump 41. It should be noted that, an assembly structure between the air nozzle 402 of the pressure sensor 42 and the second inner plug-connection port 307 is substantially the same as an assembly structure between the air nozzle 401 of the air pump 41 and the first inner plug-connection port 307, and details are not described repeatedly below.

In this embodiment, the air nozzle 401 of the blood pressure measurement assembly 40 is plug-connected to the inner plug-connection port 307, and extends in the Z direction. The air nozzle 401 of the blood pressure measurement assembly 40 is plug-connected to the inner plug-connection port 307 from the upper end portion of the inner plug-connection port 307. Because the inner diameter of the upper end portion of the inner plug-connection port 307 gradually increases in the Z direction, the air nozzle 401 of the blood pressure measurement assembly 40 can be more easily plug-connected to the inner plug-connection port 307. In addition, an inner diameter of the lower end portion of the inner plug-connection port 307 is slightly less than or equal to an outer diameter of the air nozzle 401 of the blood pressure measurement assembly 40, so as to ensure that when the air nozzle 401 of the blood pressure measurement assembly 40 extends into the lower end portion from the upper end portion of the inner plug-connection port 307, the air nozzle 401 of the blood pressure measurement assembly 40 closely adheres to the first covering member 32, thereby ensuring connection reliability between the air nozzle 401 of the blood pressure measurement assembly 40 and the first covering member 32.

It should be understood that, in another embodiment, a manner of forming the inner plug-connection port 307 may be the same as a manner of forming the outer plug-connection port 305. In this case, the top face 323 of the first covering portion 321 partially protrudes to form an inner plug-connection nozzle, and the inner plug-connection port 307 is formed on an inner side of the inner plug-connection nozzle. The air nozzle 401 of the blood pressure measurement assembly 40 and the inner plug-connection nozzle are plug-connected to each other, to be specific, the air nozzle 401 of the blood pressure measurement assembly 40 is plug-connected to the inner plug-connection port 307, or the air nozzle 401 of the blood pressure measurement assembly 40 is sleeved with the inner plug-connection nozzle.

In an implementation, an elastic modulus of the air nozzle 401 of the blood pressure measurement assembly 40 is greater than an elastic modulus of the first covering member 32. That is, the air nozzle 401 of the blood pressure measurement assembly 40 is prepared by using a rigid material, and the first covering member 32 is prepared by using an elastic material. To be specific, the air nozzle 401 of the blood pressure measurement assembly 40 is prepared by using a relatively hard material, and the first covering member 32 is prepared by using a relatively soft material. When the air nozzle 401 of the blood pressure measurement assembly 40 is inserted into the inner plug-connection port 307, the first covering member 32 deforms and closely adheres to the air nozzle 401 of the blood pressure measurement assembly 40 as being squeezed by the air nozzle 401 of the blood pressure measurement assembly 40, thereby ensuring air-tightness between the air nozzle 401 of the blood pressure measurement assembly 40 and the first covering member 32. It should be understood that, sealing may also be implemented between the air nozzle 401 of the blood pressure measurement assembly 40 and the first covering member 32 by using a structure such as a sealing ring or a sealing gasket. This is not specifically limited in this application.

The sensor assembly is spaced from the blood pressure measurement assembly 40, and is electrically connected to the processor. Specifically, the sensor assembly includes a heart rate detection sensor, and the heart rate detection sensor directly faces the mounting hole (not shown) or is at least partially accommodated in the mounting hole. The heart rate detection sensor is an optical heart rate sensor. When the wearable device 100 is worn by a user, the bottom face 312b of the boss 312a adheres to the user's wrist, and the optical heart rate sensor may send a heart rate sensing signal through the mounting hole, so as to implement accurate heart rate detection, thereby improving functional diversity of the wearable device 1000, and improving use experience of the user. Certainly, the heart rate detection sensor may alternatively be a sensor that can perform heart rate detection, such as a bone conduction sensor.

In addition, the sensor assembly may further include a sensor such as a gyroscope sensor, a barometric pressure sensor, an acceleration sensor, a distance sensor, an optical proximity sensor, a fingerprint sensor, a temperature sensor, or an ambient light sensor, to further improve functional diversity of the wearable device 1000 and improve use experience of the user.

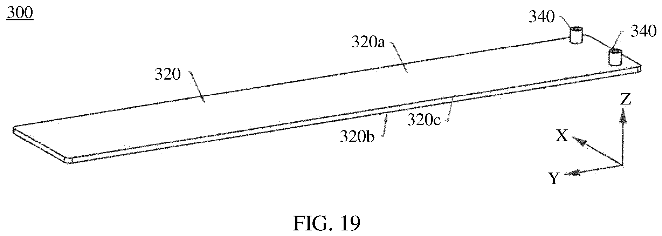

Refer to FIG. 2 and FIG. 19. FIG. 19 is a schematic structural diagram of the compression band 300 in the wearable device 1000 shown in FIG. 2.

The compression band 300 is located on one side of the watch face 100. The compression band 300 includes a cuff 320, an airbag (not shown) accommodated in the cuff 320, and an air nozzle 340 communicating with the airbag. The cuff 320 includes a top face 320a and a bottom face 320b that are oppositely disposed, and a peripheral face 320c connected between the top face 320a and the bottom face 320b. The airbag is located at a position of the cuff 320 that is close to the watch face 100. The air nozzle 340 protrudes from the top face 320a of the cuff 320. The air nozzle 340 extends from the top face 320a of the cuff 320 in a direction away from the bottom face 320b, that is, the air nozzle 340 extends in the Z direction. A material of the air nozzle 340 may be the same as that of the airbag. In this case, the air nozzle 340 may be integrally formed with the airbag. Alternatively, a material of the air nozzle 340 may be different from that of the airbag. In this case, the air nozzle 340 may be detachably formed with the airbag through assembly.

In this embodiment, there are two air nozzles 340. The two air nozzles 340 are a first air nozzle 340 and a second air nozzle 340, and the first air nozzle 340 and the second air nozzle 340 are spaced in the X direction. Structures of the first air nozzle 340 and the second air nozzle 340 are basically the same.

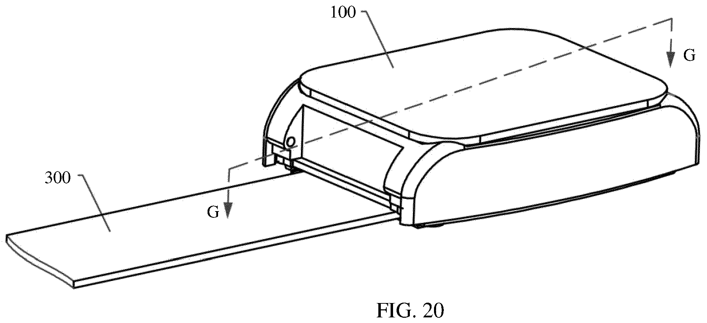

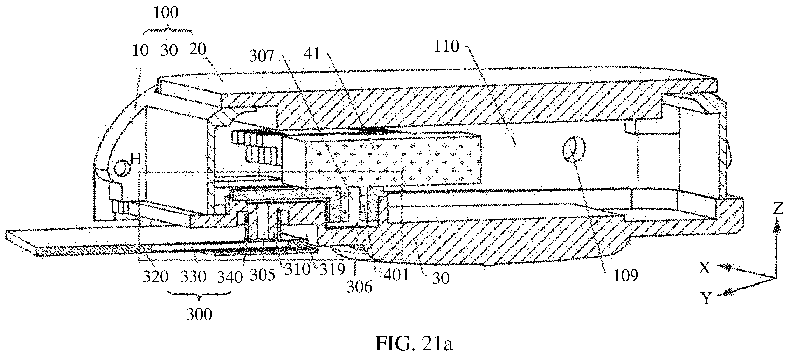

FIG. 20 is a schematic structural diagram of an assembly of the compression band 300 and the watch face 100 in the wearable device 1000 shown in FIG. 2. FIG. 21a is a schematic structural diagram of the structure shown in FIG. 20 that is sectioned in a G-G direction. Both FIG. 20 and FIG. 21a show only a part in which the compression band 300 is connected to the watch face 100.

The compression band 300 partially extends into the assembly groove 319 and is fixed to the assembly groove 319, and partially extends out of the assembly groove 319. In this case, the compression band 300 shares all of the thickness distance of the bottom cover 30 in the Z direction, so as to reduce impact on a thickness of the wearable device 1000 caused by assembling the compression band 300 on the bottom cover 30, and help implement a light and thin design of the wearable device 1000.

The air nozzle 340 of the compression band 300 is located in the assembly groove 319. Specifically, the air nozzle 340 of the compression band 300 mutually communicates with the outer plug-connection port 305. That is, the air nozzle 340 of the compression band 300 communicates with the air nozzle 401 of the blood pressure measurement assembly 40 through the flow passage 306. In this case, an airbag 330 of the compression band 300 communicates with the outer plug-connection port 305 through the air nozzle 340. That is, the airbag 330 of the compression band 300 communicates with the air nozzle 401 of the blood pressure measurement assembly 40 through the air nozzle 340 of the compression band 300 and the flow passage 305.

A cross-sectional area of the flow passage 306 is greater than or equal to 1 mm², that is, an inner diameter of the flow passage 306 is large enough, so that resistance generated when air flows inside the flow passage 306 is relatively small. This ensures that an air exchange rate between the air nozzle 340 of the compression band 300 and the air nozzle 401 of the blood pressure detection assembly 40 is high enough, facilitates fast air circulation between the air nozzle 340 of the compression band 300 and the air nozzle 401 of the blood pressure detection assembly 40, and improves blood pressure detection efficiency of the wearable device 1000.

In the wearable device 1000 illustrated in this embodiment, the flow passage 306 isolates the air nozzle 340 of the compression band 300 from the air nozzle 401 of the blood pressure measurement assembly 40, and the blood pressure measurement assembly 40 is not easily affected and displaced in a process of assembling or disassembling the compression band 300, so as to avoid a problem that the blood pressure measurement assembly 40 is damaged when the compression band 300 is moved, thereby helping prolonging a service life of the wearable device 1000.

In addition, the outer plug-connection port 305, the flow passage 306, and the inner plug-connection port 307 are all integrated in the bottom cover 30 of the watch face 100, and there is no need to dispose a communication pipeline, in the watch face inner cavity 110, that communicates between the air nozzle 340 of the compression band 300 and the air nozzle 340 of the blood pressure measurement assembly 40. This avoids space usage of the communication pipeline in the watch face inner cavity 110, helps increase space utilization of the watch face inner cavity 110, implements a light and thin design of the watch face 100, and improves appearance exquisiteness of the wearable device 1000.

Moreover, the outer plug-connection port 305 communicating with the air nozzle 340 of the compression band 300 is closer to the bezel 10, that is, the inner plug-connection port 307 communicating with the air nozzle 401 of the blood pressure measurement assembly 40 is closer to the middle portion of the bottom cover 30. In this way, the air nozzle 340 of the compression band 300 can communicate with the air nozzle 401 of the blood pressure measurement assembly 40 without extending to directly under the blood pressure measurement assembly 40. An extension length of the compression band 300 under the watch face 100 can be reduced, and this helps reduce an overall thickness of the wearable device 1000 and implement a light and thin design of the wearable device 1000. Further, a position between the compression band 300 and the blood pressure measurement assembly 40 can be more flexibly designed, and positions of functional components located in the watch face inner cavity 110 such as a battery and/or a sensor module can be more flexibly designed. This helps increase the space utilization of the watch face inner cavity 110 and implement a light and thin design of the wearable device 1000.

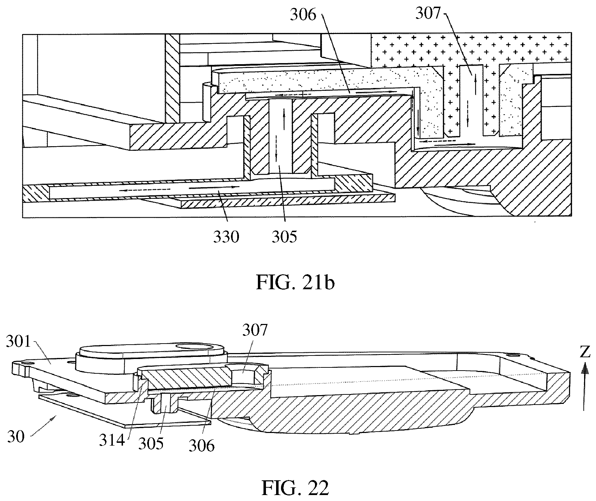

Refer to FIG. 21a and FIG. 21b. FIG. 21b is an enlarged schematic structural diagram of region H in the structure shown in FIG. 21a.

In this embodiment, the first air nozzle 340 of the compression band 300 mutually communicates with the first outer plug-connection port 305. In this case, the first air nozzle 340 of the compression band 300 communicates with the air nozzle 401 of the air pump 41 through the first flow passage 306. When the wearable device 1000 needs to perform blood pressure measurement, the air pump 41 may deliver outside air sucked through the breather hole 109 to the airbag 330 of the compression band 300 by using the first flow passage 306 and the air nozzle 340 of the compression band 300, so as to inflate the airbag 330 of the compression band 300. When the wearable device 1000 has finished blood pressure measurement, air in the airbag 330 of the compression band 300 may be delivered to the air pump 41 through the air nozzle 340 of the compression band 300 and the first flow passage 306, and the air pump 41 may discharge the air through the breather hole 109. When the air pump 41 inflates the airbag 330, a air flow path is shown by a dashed arrow in FIG. 21b. When air in the airbag 330 is discharged by the air pump 41, an air flow path is shown by a solid arrow in FIG. 21b. Description of a solid arrow and a dashed arrow in the following accompany figures is understood in a same way.

The second air nozzle 340 of the compression band 300 mutually communicates with the second outer plug-connection port 305. In this case, the second air nozzle 340 of the compression band 300 communicates with the air nozzle 401 of the pressure sensor 42 through the second flow passage 306. The pressure sensor 42 may sense a pressure value inside the airbag 330 of the compression band 300 through the second flow passage 306.

In the wearable device 1000 illustrated in this embodiment, the air nozzles 401 of the air pump 41 and the pressure sensor 42 respectively communicate with the air nozzles 340 of the compression band 300 through the two flow passages 306, instead of sharing one flow passage. This not only can improve flexibility of mounting the blood pressure measurement assembly 40 in the watch face inner cavity 110, but also can prevent air transmission between the air pump 41 and the airbag 330 of the compression band 300 and air transmission between the pressure sensor 42 and the airbag 330 of the compression band 300 from interfering with each other, thereby helping improve measurement sensitivity and precision of the blood pressure measurement assembly 40 in a blood pressure measurement process.

The following provides description by using an assembly structure between the first air nozzle 340 of the compression band 30 and the first outer plug-connection nozzle 310 as an example. An air nozzle 340 of the compression band 300 mentioned below refers to the first air nozzle 340 of the compression band 340, and an outer plug-connection nozzle 310 mentioned below refers to the first outer plug-connection nozzle 310. It should be understood that, an assembly structure between the second air nozzle 340 of the compression band 300 and the second outer plug-connection nozzle 310 is basically the same as the assembly structure between the first air nozzle 340 and the first outer plug-connection nozzle 310, and details are not described repeatedly below.

Specifically, the air nozzle 340 of the compression band 300 is sleeved with the outer plug-connection nozzle 310, that is, the outer plug-connection nozzle 310 is plug-connected to the air nozzle 340 of the compression band 300, so that the compression band 300 and the bottom cover 30 are connected to each other. An inner diameter of the air nozzle 340 of the compression band 300 is slightly smaller than an outer diameter of the outer plug-connection nozzle 310, so that when the air nozzle 340 of the compression band 300 is sleeved with the outer plug-connection nozzle 310, that is, when the outer plug-connection nozzle 310 is plug-connected to the air nozzle 340 of the compression band 300, the air nozzle 340 of the compression band 300 can closely adhere to the outer plug-connection nozzle 310, thereby ensuring connection reliability between the air nozzle 340 of the compression band 300 and the outer plug-connection nozzle 310.

It can be understood that, compared with the embodiment in which the second distance range [Z21, Z22] totally overlaps the first distance range [Z11, Z12], in the wearable device 1000 illustrated in this embodiment, in the Z direction, a length size of the outer plug-connection nozzle 310 is larger, an area of an outer peripheral face of the outer plug-connection nozzle 310 is also larger, and a contact area between the air nozzle 340 of the compression band 300 and the outer plug-connection nozzle 310 is also larger. This not only improves connection reliability between the air nozzle 340 of the compression band 300 and the outer plug-connection nozzle 310, but also reduces a risk of air leakage between the air nozzle 340 of the compression band 300 and the outer plug-connection nozzle 310.

In an implementation, an elastic modulus of the main body member 31 is greater than an elastic modulus of the air nozzle 340 of the compression band 300. That is, the main body member 31 is prepared by using a rigid material, and the air nozzle 340 of the compression band 300 is prepared by using an elastic material. To be specific, the outer plug-connection nozzle 310 of the main body member 31 is prepared by using a relatively hard material, and the air nozzle 340 of the compression band 300 is prepared by using a relatively soft material. When the outer plug-connection nozzle 310 is inserted into the air nozzle 340 of the compression band 300, because the outer plug-connection nozzle 310 is relatively hard and the air nozzle 340 of the compression band 300 is relatively soft, the air nozzle 340 of the compression band 300 deforms and closely adheres to the outer plug-connection nozzle 310 as being squeezed by the outer plug-connection nozzle 310, thereby ensuring air-tightness between the outer plug-connection nozzle 310 and the air nozzle 340 of the compression band 300. It can be understood that, sealing may also be implemented between the air nozzle 340 of the compression band 300 and the outer plug-connection nozzle 310 by using a manner of a sealing ring or a sealing gasket. This is not specifically limited in this application.

The air nozzle 340 of the compression band 300 partially extends into the avoidance groove 3191. An inner diameter of the avoidance groove 3191 is greater than or equal to an outer diameter of the air nozzle 340 of the compression band 300, so that the air nozzle 340 of the compression band 300 is inserted into the avoidance groove 3191 and is avoided, to reduce impact of the assembly structure between the air nozzle 340 of the compression band 300 and the outer plug-connection nozzle 310 on the thickness of the wearable device 1000, thereby helping implement a light and thin design of the wearable device 1000. It should be understood that, the air nozzle 340 of the compression band 300 may be totally accommodated in the avoidance groove 3191. In this case, the outer plug-connection nozzle 310 only needs to be kept from extending to protrude from the bottom groove wall of the assembly groove 319. This can further reduce the impact of the assembly structure between the air nozzle 340 of the compression band 300 and the outer plug-connection nozzle 310 on the thickness of the wearable device 1000.

The fixing member 33 is fixed to a side of the compression band 300 that is away from the main body member 31, and abuts against the compression band 300. This not only prevents the air nozzle 340 of the compression band 300 from falling off the outer plug-connection nozzle 310 and maintains plug-connection stability between the air nozzle 340 of the compression band 300 and the outer plug-connection nozzle 310, but also prevents the compression band 300 from falling off the assembly groove 319 and improves assembly stability between the compression band 300 and the bottom cover 30.

In the wearable device 1000 illustrated in this embodiment, the blood pressure measurement assembly 40 is integrated into the watch face 100, and the compression band 300 stacked with the watch band 200 is added. When the wearable device 1000 is worn on a user's wrist, the user may perform blood pressure measurement anytime anywhere. A blood pressure status of the user can be learned in time, without using an additional sphygmomanometer for blood pressure measurement, thereby improving user convenience in blood pressure measurement, and improving use experience of the user.

When the wearable device 1000 is worn on a user's wrist, the compression band 300 adheres to the user's wrist. When the wearable device 1000 receives input of a blood pressure measurement instruction, the processor sends a blood pressure measurement signal to the air pump 41 and the pressure sensor 42. The air pump 41 sucks air in an outside environment from the breather hole 109, and inflates and pressurizes the airbag 330 of the compression band 300 through the first flow passage 306 and the first air nozzle 340 of the compression band 300. In this case, the compression band 300 bulges and compresses the user's wrist artery. The pressure sensor 42 detects a pressure value in the airbag 330 of the compression band 300 by using the second flow passage 306 and the second air nozzle 340 of the compression band 300, and feeds back the pressure value to the processor in real time. The processor determines whether the pressure value in the airbag 330 of the compression band 300 meets a requirement for blood pressure measurement. If the requirement is not met, the processor continues to send the blood pressure measurement signal to the air pump 41, and the air pump 41 continues to inflate and pressurize the airbag 330 of the compression band 300, until the processor determines that the pressure value fed back by the pressure sensor 42 meets the requirement for blood pressure measurement. The processor calculates the user's blood pressure based on the pressure value fed back by the pressure sensor 42 in real time, and displays and feeds back the blood pressure to the user by using the display. After the blood pressure measurement is complete, the processor stops sending the blood pressure measurement signal, and the air pump 41 and the pressure sensor 42 both stop working. A air in the airbag 330 of the compression band 300 is delivered to the air pump 41 through the first air nozzle 340 of the compression band 300 and the first flow passage 306. The air pump 41 discharges the air through the breather hole 109. At this time, the compression band 300 is restored to be flat and adheres to the user's wrist.

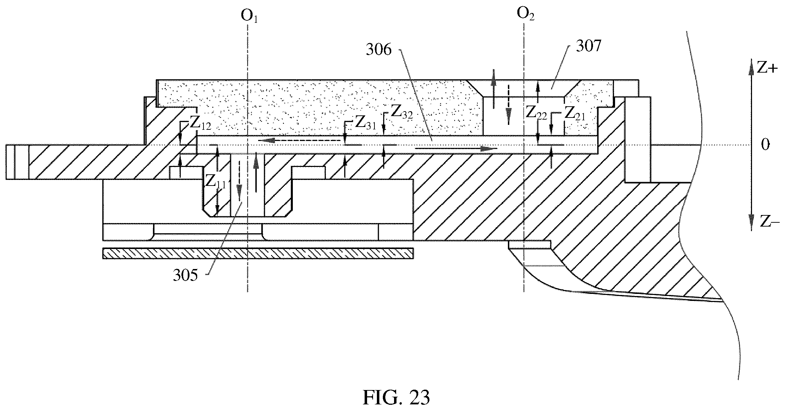

FIG. 22 is a schematic cross-sectional structural diagram of a bottom cover 30 in a C-C direction in a second wearable device 1000 according to an embodiment of this application. FIG. 23 is a partial schematic structural diagram of a front view of the structure shown in FIG. 22.

A difference between the wearable device 1000 illustrated in this embodiment and the wearable device 1000 illustrated in the foregoing embodiment lies in that the outer plug-connection port 305 and the inner plug-connection port 307 are respectively located on two opposite sides of the top face 301 of the bottom cover 30. Specifically, the outer plug-connection port 305 is located under the top face 301 of the bottom cover 30, and a first distance range [Z11, Z12] is formed between the outer plug-connection port 305 and the top face 301 of the bottom cover 30, where Z11 < Z12 < 0. The inner plug-connection port 307 is located above the top face 301 of the bottom cover 30, and a second distance range [Z21, Z22] is formed between the inner plug-connection port 307 and the top face 301 of the bottom cover, where 0 < Z21 < Z22.

In this case, the first distance range [Z11, Z12] does not overlap the second distance range [Z21, Z22]. That is, the outer plug-connection port 305 and the inner plug-connection port 307 are completely staggered in the direction from the central axis O1 of the outer plug-connection port 305 to the central axis O2 of the inner plug-connection port 307. In other words, the outer plug-connection port 305 and the inner plug-connection port 307 do not share a thickness distance of the bottom cover 30 in the Z direction.

The flow passage 306 is hyphen-shaped and parallel to the X-Y plane. A third distance range [Z31, Z32] is formed between the flow passage 306 and the top face 301 of the bottom cover 30. Specifically, the lower end face of the flow passage 306 is flush with the upper port of the outer plug-connection port 305, that is, Z31 = Z12. The upper end face of the flow passage 306 is flush with the lower port of the inner plug-connection port 307, that is, Z31 = Z22. In this case, only endpoints in the third distance range [Z31, Z32] are located in a combination set of the second distance range [Z21, Z22] and the first distance range [Z11, Z12]. That is, the flow passage 306 is staggered from both the outer plug-connection port 305 and the inner plug-connection port 306 in the direction from the central axis O1 of the outer plug-connection port 305 to the central axis O2 of the inner plug-connection port 307. It can be understood that, in the wearable device 1000 illustrated in this embodiment, structures of the outer plug-connection port 305, the flow passage 306, and the inner plug-connection port 306 are simple. This simplifies a process of forming the bottom cover 30, and further reduces a preparation cost of the bottom cover 30.

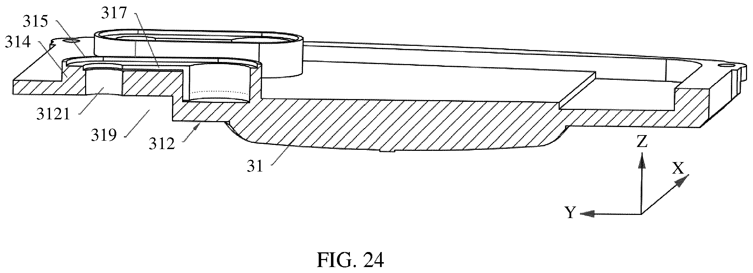

FIG. 24 is a schematic structural diagram of the main body member 31 that is sectioned in a D-D direction in the bottom cover 30 in a third wearable device 1000 according to an embodiment of this application.

A difference between the wearable device 1000 provided in this embodiment of this application and the wearable devices 1000 illustrated in the foregoing two embodiments lies in that the bottom face 312 of the main body member 31 is partially recessed to form a second mounting groove 3121, and the second mounting groove 3121 communicates with the flow groove 317. Specifically, the bottom groove wall of the assembly groove 319 is partially recessed to form the second mounting groove 3121. That is, an opening of the second mounting groove 3121 is located on the bottom groove wall of the assembly groove 319. The second mounting groove 3121 is recessed in a direction from the bottom groove wall of the assembly groove 319 to the top face 315 of the boss 314, and runs through the bottom groove wall of the flow groove 317.

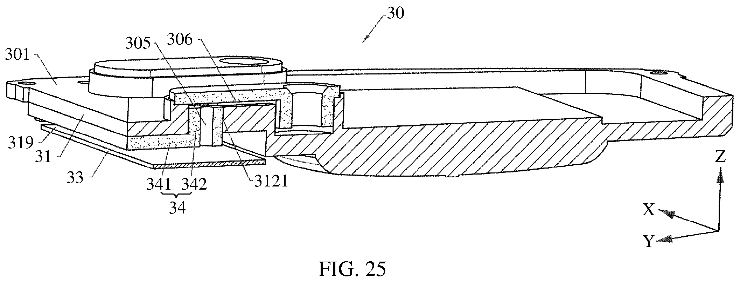

FIG. 25 is a schematic structural diagram of the bottom cover 30 that is sectioned in a C-C direction in the third wearable device 1000 according to an embodiment of this application.

In this embodiment, the bottom cover 30 further includes a second covering member 34, the second covering member 34 is located on a side of the main body member 31 that is away from the first covering member 32, and the second covering member 34 forms the outer plug-connection port 305. The second covering member 34 includes a second covering portion 341 and a second plug-connection portion 342 connected to the second covering portion 341. The first covering portion 321 has a substantially flat plate structure. The second covering portion 341 includes a top face and a bottom face that are oppositely disposed. The second plug-connection portion 342 is connected to the top face of the second covering portion 341. The second plug-connection portion 342 extends from the top face of the second covering portion 341 in a direction away from the bottom face, and has a substantially circular tubular structure. The second plug-connection portion 342 includes a top face that has a same orientation as the top face of the second covering portion 341. The top face of the second plug-connection portion 342 is partially recessed to form the outer plug-connection port 305, that is, an opening of the outer plug-connection port 305 is located on the top face of the second plug-connection portion 342. The outer plug-connection port 305 extends in a direction from the top face of the second plug-connection portion 342 to the bottom face of the second covering portion 341, and runs through the bottom face of the second covering portion 341. The second covering member 34 is prepared by using an elastic material such as silica gel or TPU.

A second fixing member is fixed to the side of the main body member 31 that is away from the first covering member 32. Specifically, the second covering member 34 is fixed to the assembly groove 319, and is located between the main body member 31 and the fixing member 33. The second covering member 34 may be detachably mounted to the assembly groove 319. The second covering portion 341 of the second covering member 34 is fixed to the assembly groove 319, and the second plug-connection portion 342 extends into the second mounting groove 3121, so that the outer plug-connection port 305 communicates with the flow passage 306.

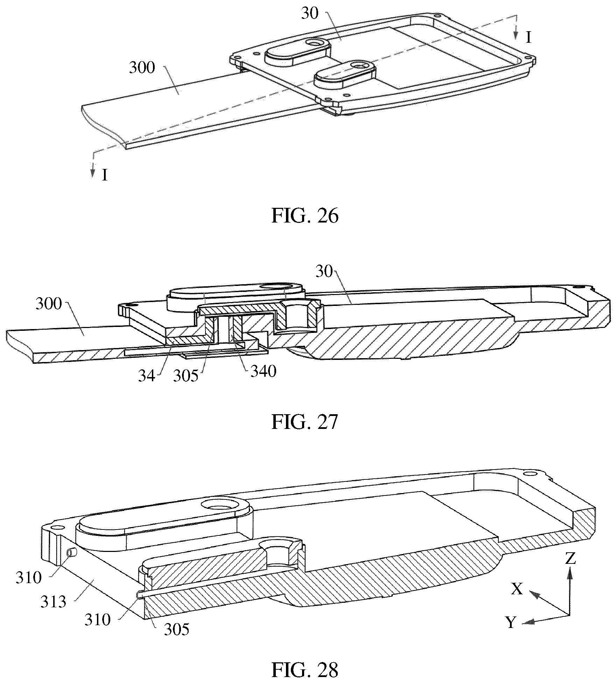

FIG. 26 is a schematic structural diagram of an assembly of the bottom cover 30 and the compression band 300 in the third wearable device 1000 according to an embodiment of this application. FIG. 27 is a schematic structural diagram of the structure shown in FIG. 26 that is sectioned in an I-I direction.

The air nozzle 340 of the compression band 300 is plug-connected to the outer plug-connection port 305, to implement mutual communication with the outer plug-connection port 305. The outer diameter of the air nozzle 340 of the compression band 300 is slightly greater than an inner diameter of the outer plug-connection port 305, to ensure that the air nozzle 340 of the compression band 300 and the second covering member 34 closely adhere to each other, and to ensure connection reliability between the air nozzle 340 of the compression band 300 and the second covering member 34.

In an implementation, an elastic modulus of the air nozzle 340 of the compression band 300 is greater than an elastic modulus of the second covering member 34. That is, the air nozzle 340 of the compression band 300 is prepared by using a rigid material, and the second covering member 34 is prepared by using an elastic material. To be specific, the air nozzle 340 of the compression band 300 is prepared by using a relatively hard material, and the second covering member 34 is prepared by using a relatively soft material. When the air nozzle 340 of the compression band 300 is inserted into the outer plug-connection port 305, the second covering member 34 deforms and closely adheres to the air nozzle 340 of the compression band 300 as being squeezed by the air nozzle 340 of the compression band 300, thereby ensuring air-tightness between the air nozzle 340 of the compression band 300 and the second covering member 34.

In the wearable device 1000 illustrated in this embodiment, the second covering member 34 is used to form the outer plug-connection port 305, and the air nozzle 340 of the compression band 300 is plug-connected to the outer plug-connection port 305, to implement mutual plug-connection to the outer plug-connection port 305. Compared with the embodiment in which the air nozzle 340 of the compression band 300 is sleeved with the outer plug-connection nozzle 310, this embodiment avoids a problem that the air nozzle 340 of the compression band 300 cannot be plug-connected to the outer plug-connection nozzle 310 due to a processing error of the air nozzle 340 of the compression band 300. This reduces a requirement on processing precision of the air nozzle 340 of the compression band 300, helps reduce a preparation cost of the compression band 300, and improves product competitiveness of the wearable device 1000.

FIG. 28 is a schematic structural diagram of the bottom cover 30 that is sectioned in a C-C direction in a fourth wearable device 1000 according to an embodiment of this application.

A difference between the wearable device 1000 illustrated in this embodiment of this application and the wearable device 1000 illustrated in the foregoing second embodiment lies in that the peripheral face 313 of the main body member 31 partially protrudes to form the outer plug-connection nozzle 310, and the outer plug-connection port 305 is formed on the inner side of the outer plug-connection nozzle 310. The outer plug-connection nozzle 310 extends from the peripheral face 313 of the main body member 31 along the X-Y plane.

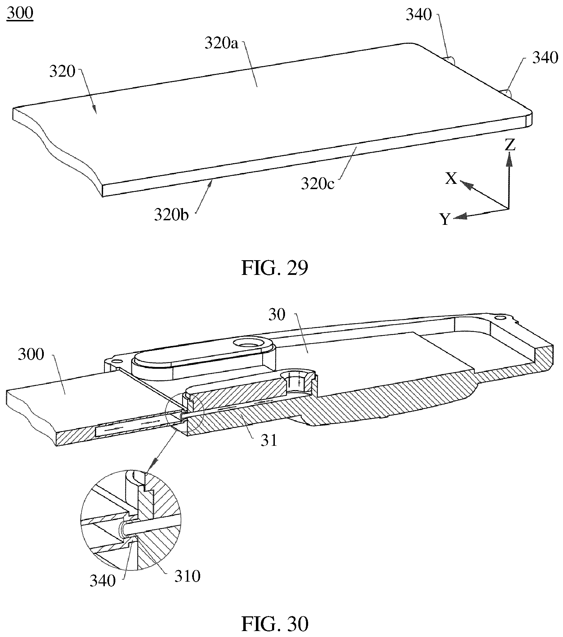

FIG. 29 is a schematic structural diagram of the compression band 300 in the fourth wearable device 1000 according to an embodiment of this application. FIG. 30 is a schematic structural diagram of an assembly structure of the compression band 300 and the bottom cover 30 that is sectioned in an I-I direction in the fourth wearable device 1000 according to an embodiment of this application.

The air nozzle 340 of the compression band 300 is sleeved with the outer plug-connection nozzle 310, that is, the outer plug-connection nozzle 310 is plug-connected to the air nozzle of the compression band 300. The air nozzle 340 of the compression band 300 protrudes from the peripheral face 320c of the cuff 320. The air nozzle 340 of the compression band 300 extends from the peripheral face 320 of the cuff 320 along the X-Y plane.

In the wearable device 1000 illustrated in this embodiment, the compression band 300 is connected to the peripheral face 313 of the main body member 31. In this case, the compression band 300 occupies no bottom face space of the bottom cover 30, and only shares a part or all of the thickness distance of the bottom cover 30 in the Z direction, so as to reduce impact of presence of the compression band 300 on the thickness of the wearable device 1000, and facilitate a light and thin design of the wearable device 1000.

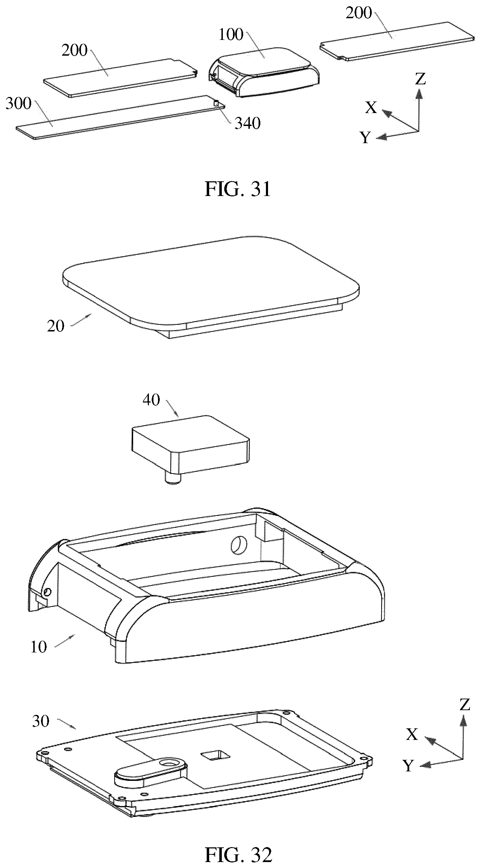

FIG. 31 is a schematic exploded structural diagram of a fifth wearable device 1000 according to an embodiment of this application. FIG. 32 is a schematic exploded structural diagram of the watch face 100 in the wearable device 1000 shown in FIG. 31.

A difference between the wearable device 100 illustrated in this embodiment and the foregoing four wearable devices 1000 lies in that the blood pressure measurement component 40 is a component that integrates functions of an air pump and a pressure sensor, that is, both the air pump and the pressure sensor are integrated into the blood pressure measurement component 40, to simply a structure of the blood pressure measurement assembly 40, reduce a volume occupied by the blood pressure measurement assembly 40 in the watch face inner cavity, and facilitate a light and thin design of the watch face 200. There are one outer plug-connection port, one flow passage, and one inner plug-connection port. An air nozzle of the blood pressure measurement component 40 mutually communicates with the inner plug-connection port, and the air nozzle 340 of the compression band 300 mutually communicates with the inner plug-connection port. In this case, there is also one air nozzle 340 of the compression band 300.

It should be understood that, in the wearable device 1000 illustrated in this embodiment, components such as the bezel 10 and the top cover 20 of the watch face 100 and other structures of the watch band 200 and the compression band 300 are basically the same as the structures of the wearable devices 1000 illustrated in the foregoing four embodiments. Details are not described herein.

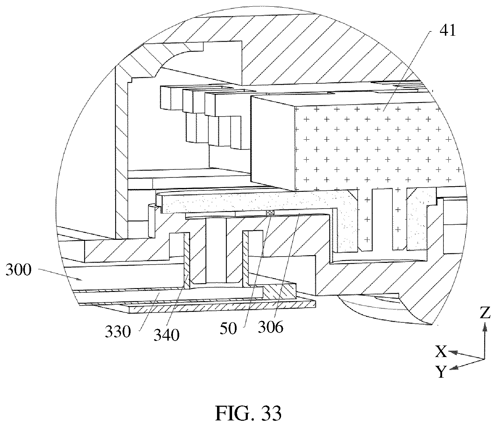

FIG. 33 is a partial schematic structural diagram of an assembly structure of the watch face 100 and the compression band 300 that is sectioned in a G-G direction in a sixth wearable device according to an embodiment of this application.

A difference between the wearable device 100 illustrated in this embodiment and the foregoing five wearable devices 100 lies in that the sensor assembly further includes a air sensor 50, and the air sensor 50 is located in the flow passage 306. Specifically, the air sensor 50 is fixed to an inner wall of the flow passage 306 and is electrically connected to the processor, so as to analyze a air component such as formaldehyde, oxygen, or carbon dioxide in an ambient environment of the wearable device 1000 when the wearable device 1000 performs blood pressure measurement, thereby improving functional diversity of the wearable device 1000 and improving use experience of a user.

Specifically, when the wearable device 1000 performs blood pressure measurement, the air pump 41 sucks a air from the surrounding environment, and fills the airbag 330 of the compression band 300 with the air through the flow passage 306 and the air nozzle 340 of the compression band 300 to perform blood pressure measurement. Because the air sensor 50 is located inside the flow passage 306, the air flows through the air sensor 50. When the pressure sensor measures a pressure value P in the airbag 33 of the compression band 300 at a specific moment, the air sensor 50 simultaneously analyzes a concentration value C of a air that needs to be measured. The processor receives the pressure value P fed back by the pressure sensor and the air concentration value C fed back by the air sensor 50, and performs processing on the pressure value P and the concentration value C to obtain a concentration of the air measured in the environment.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A wearable device (1000), comprising a watch face (100), a watch band (200), and a compression band (300), wherein the watch face (100) comprises a bezel (10), a bottom cover (30), and a blood pressure measurement assembly (40), the bezel (10) is connected to a peripheral edge of the bottom cover (30) and encloses a watch face inner cavity (110) together with the bottom cover (30),
wherein the bottom cover (30) is provided with an outer plug-connection port (305), a flow passage (306), and an inner plug-connection port (307) that successively communicate, the outer plug-connection port (305) is closer to the bezel (10) than the inner plug-connection port (307), the blood pressure measurement assembly (40) is accommodated in the watch face inner cavity (110), and an air nozzle of the blood pressure measurement assembly (40) mutually communicates with the inner plug-connection port (307); and
the watch band (200) is connected to the bezel (10), the compression band (300) and the watch band (200) are stacked, and an air nozzle of the compression band (300) mutually communicates with the outer plug-connection port (305);
wherein the outer plug-connection port (305), the flow passage (306), and the inner plug-connection port (307) are all integrated in the bottom cover (30) of the watch face (100).

2. The wearable device (1000) according to claim 1, wherein the bottom cover (30) comprises a top face (301) that faces the watch face inner cavity (110), a distance between the outer plug-connection port (305) and the top face (301) of the bottom cover (30) forms a first distance range, a distance between the inner plug-connection port (307) and the top face (301) of the bottom cover (30) forms a second distance range, and the second distance range partially or totally overlaps the first distance range.

3. The wearable device (1000) according to claim 1 or 2, wherein the flow passage (306) is hyphen-shaped, S-shaped, L-shaped, or Z-shaped.

4. The wearable device (1000) according to any one of claims 1 to 3, wherein the bottom cover (30) comprises a main body member (31) and a first covering member (32), a peripheral edge of the main body member (31) is connected to the bezel (10), the first covering member (32) is fixed to one side of the main body member (31), and the first covering member (32) forms the inner plug-connection port (307) and forms the flow passage (306) through enclosure together with the main body member (31).

5. The wearable device (1000) according to claim 4, wherein the main body member (31) comprises a top face (301) that faces the watch face inner cavity (110), the top face (301) of the main body member (31) partially protrudes to form a boss, and the first covering member (32) is fixedly connected to the boss and forms the flow passage (306) through enclosure together with the boss.

6. The wearable device (1000) according to claim 4 or 5, wherein the air nozzle of the blood pressure measurement assembly (40) is plug-connected to the inner plug-connection port (307), and an elastic modulus of the air nozzle of the blood pressure measurement assembly (40) is greater than an elastic modulus of the first covering member (32).

7. The wearable device (1000) according to any one of claims 4 to 6, wherein the bottom cover (30) further comprises a second covering member (34), the second covering member (34) is fixed to a side of the main body member (31) that is away from the first covering member (32), the second covering member (34) forms the outer plug-connection port (305), the air nozzle of the compression band (300) is plug-connected to the outer plug-connection port (305), and an elastic modulus of the air nozzle of the compression band (300) is greater than an elastic modulus of the second covering member (34).

8. The wearable device (1000) according to any one of claims 4 to 6, wherein the main body member (31) comprises a bottom face that is away from the watch face inner cavity (110), the bottom face of the main body member (31) is partially recessed to form an assembly groove (319), a groove wall of the assembly groove (319) partially protrudes to form an outer plug-connection nozzle (310), the outer plug-connection port (305) is formed on an inner side of the outer plug-connection nozzle (310), the air nozzle of the compression band (300) is located in the assembly groove (319), the outer plug-connection nozzle (310) is plug-connected to the air nozzle of the compression band (300), and an elastic modulus of the main body member (31) is greater than an elastic modulus of the air nozzle of the compression band (300).

9. The wearable device (1000) according to claim 8, wherein the outer plug-connection nozzle (310) is located between the top face (301) of the main body member (31) and the bottom face of the main body member (31).

10. The wearable device (1000) according to any one of claims 4 to 6, wherein a peripheral face of the main body member (31) partially protrudes to form an outer plug-connection nozzle (310), the outer plug-connection port (305) is formed on an inner side of the outer plug-connection nozzle (310), the air nozzle of the compression band (300) is sleeved with the outer plug-connection nozzle (310), and an elastic modulus of the air nozzle of the compression band (300) is less than an elastic modulus of the main body member (31).

11. The wearable device (1000) according to any one of claims 1 to 10, wherein the wearable device (1000) further comprises an air sensor (50), and the air sensor (50) is located in the flow passage (306) and fixed to an inner wall of the flow passage (306).

12. The wearable device (1000) according to any one of claims 1 to 11, wherein there are two outer plug-connection ports (305), two flow passages (306), and two inner plug-connection ports (307), the two outer plug-connection ports (305) are a first outer plug-connection port (305) and a second outer plug-connection port (305), the two flow passages (306) are a first flow passage (306) and a second flow passage (306), the two inner plug-connection ports (307) are a first inner plug-connection port (307) and a second inner plug-connection port (307), the first outer plug-connection port (305), the first flow passage (306), and the first inner plug-connection port (307) successively communicate, and the second outer plug-connection port (305), the second flow passage (306), and the second inner plug-connection port (307) successively communicate;
the blood pressure measurement assembly (40) comprises a pressure sensor and an air pump, a air nozzle of the pressure sensor mutually communicates with the first inner plug-connection port (307), and a air nozzle of the air pump mutually communicates with the second inner plug-connection port (307); and
the compression band (300) comprises two air nozzles, one air nozzle communicates with the first outer plug-connection port (305), and the other air nozzle communicates with the second outer plug-connection port (305).

13. The wearable device (1000) according to claim 12, wherein the bezel (10) or the bottom cover (30) is provided with a breather hole (109), and the breather hole (109) communicates with the watch face inner cavity (110) and an outside of the watch face (100).

14. The wearable device (1000) according to any one of claims 1 to 13, wherein the wearable device (1000) further comprises a heart rate detection sensor, a middle portion of the bottom cover (30) is provided with a mounting hole spaced from the inner plug-connection port (307), and the heart rate detection sensor is accommodated in the watch face inner cavity (110), and directly faces the mounting hole or is at least partially accommodated in the mounting hole.

## Patentansprüche

1. Am Körper tragbare Vorrichtung (1000), umfassend ein Ziffernblatt (100), ein Uhrenarmband (200) und ein Kompressionsband (300), wobei das Ziffernblatt (100) eine Lünette (10), eine untere Abdeckung (30) und eine Blutdruckmessbaugruppe (40) umfasst, wobei die Lünette (10) mit einer Umfangskante der unteren Abdeckung (30) verbunden ist und zusammen mit der unteren Abdeckung (30) einen inneren Hohlraum (110) des Ziffernblatts umschließt,
wobei die untere Abdeckung (30) mit einer äußeren Steckverbindungsöffnung (305), einem Strömungsdurchgang (306) und einer inneren Steckverbindungsöffnung (307) bereitgestellt ist, die nacheinander miteinander in gegenseitiger Verbindung stehen, die äußere Steckverbindungsöffnung (305) näher an der Lünette (10) ist als die innere Steckverbindungsöffnung (307), die Blutdruckmessbaugruppe (40) in dem inneren Hohlraum (110) des Ziffernblatts untergebracht ist und eine Luftdüse der Blutdruckmessbaugruppe (40) mit der inneren Steckverbindungsöffnung (307) in gegenseitiger Verbindung steht; und
das Uhrenarmband (200) mit der Lünette (10) verbunden ist, das Kompressionsband (300) und das Uhrenarmband (200) gestapelt sind und eine Luftdüse des Kompressionsbandes (300) mit der äußeren Steckverbindungsöffnung (305) in gegenseitiger Verbindung steht;
wobei die äußere Steckverbindungsöffnung (305), der Strömungsdurchgang (306) und die innere Steckverbindungsöffnung (307) alle in die untere Abdeckung (30) des Ziffernblatts (100) integriert sind.

2. Am Körper tragbare Vorrichtung (1000) nach Anspruch 1, wobei die untere Abdeckung (30) eine obere Fläche (301) umfasst, die dem inneren Hohlraum (110) des Ziffernblatts zugewandt ist, ein Abstand zwischen der äußeren Steckverbindungsöffnung (305) und der oberen Fläche (301) der unteren Abdeckung (30) einen ersten Abstandsbereich bildet, ein Abstand zwischen der inneren Steckverbindungsöffnung (307) und der oberen Fläche (301) der unteren Abdeckung (30) einen zweiten Abstandsbereich bildet und der zweite Abstandsbereich den ersten Abstandsbereich teilweise oder vollständig überlappt.

3. Am Körper tragbare Vorrichtung (1000) nach Anspruch 1 oder 2, wobei der Strömungsdurchgang (306) hyphenförmig, S-förmig, L-förmig oder Z-förmig ist.

4. Am Körper tragbare Vorrichtung (1000) nach einem der Ansprüche 1 bis 3, wobei die untere Abdeckung (30) ein Hauptkörperelement (31) und ein erstes Abdeckungselement (32) umfasst, eine Umfangskante des Hauptkörperelements (31) mit der Lünette (10) verbunden ist, das erste Abdeckungselement (32) an einer Seite des Hauptkörperelements (31) befestigt ist und das erste Abdeckungselement (32) die innere Steckverbindungsöffnung (307) bildet und zusammen mit dem Hauptkörperelement (31) den Strömungsdurchgang (306) durch ein Gehäuse bildet.

5. Am Körper tragbare Vorrichtung (1000) nach Anspruch 4, wobei das Hauptkörperelement (31) eine obere Fläche (301) umfasst, die dem inneren Hohlraum (110) des Ziffernblatts zugewandt ist, die obere Fläche (301) des Hauptkörperelements (31) teilweise vorsteht, um einen Vorsprung zu bilden, und das erste Abdeckelement (32) fest mit dem Vorsprung verbunden ist und zusammen mit dem Vorsprung den Strömungsdurchgang (306) durch ein Gehäuse bildet.

6. Am Körper tragbare Vorrichtung (1000) nach Anspruch 4 oder 5, wobei die Luftdüse der Blutdruckmessbaugruppe (40) mit der inneren Steckverbindungsöffnung (307) steckverbunden ist und ein Elastizitätsmodul der Luftdüse der Blutdruckmessbaugruppe (40) größer ist als ein Elastizitätsmodul des ersten Abdeckelements (32).

7. Am Körper tragbare Vorrichtung (1000) nach einem der Ansprüche 4 bis 6, wobei die untere Abdeckung (30) ferner ein zweites Abdeckelement (34) umfasst, das zweite Abdeckelement (34) an einer von dem ersten Abdeckelement (32) abgewandten Seite des Hauptkörperelements (31) befestigt ist, das zweite Abdeckelement (34) die äußere Steckverbindungsöffnung (305) bildet, die Luftdüse des Kompressionsbandes (300) mit der äußeren Steckverbindungsöffnung (305) steckverbunden ist und ein Elastizitätsmodul der Luftdüse des Kompressionsbandes (300) größer ist als ein Elastizitätsmodul des zweiten Abdeckelements (34).

8. Am Körper tragbare Vorrichtung (1000) nach einem der Ansprüche 4 bis 6, wobei das Hauptkörperelement (31) eine untere Fläche umfasst, die von dem inneren Hohlraum (110) des Ziffernblatts entfernt ist, die untere Fläche des Hauptkörperelements (31) teilweise vertieft ist, um eine Baugruppennut (319) zu bilden, eine Nutwand der Baugruppennut (319) teilweise vorsteht, um eine äußere Steckverbindungsdüse (310) zu bilden, die äußere Steckverbindungsöffnung (305) an einer Innenseite der äußeren Steckverbindungsdüse (310) gebildet ist, die Luftdüse des Kompressionsbandes (300) in der Baugruppennut (319) lokalisiert ist, die äußere Steckverbindungsdüse (310) mit der Luftdüse des Kompressionsbandes (300) steckverbunden ist und ein Elastizitätsmodul des Hauptkörperelements (31) größer ist als ein Elastizitätsmodul der Luftdüse des Kompressionsbandes (300).

9. Am Körper tragbare Vorrichtung (1000) nach Anspruch 8, wobei die äußere Steckverbindungsdüse (310) zwischen der oberen Fläche (301) des Hauptkörperelements (31) und der unteren Fläche des Hauptkörperelements (31) lokalisiert ist.

10. Am Körper tragbare Vorrichtung (1000) nach einem der Ansprüche 4 bis 6, wobei eine Umfangsfläche des Hauptkörperelements (31) teilweise vorsteht, um eine äußere Steckverbindungsdüse (310) zu bilden, die äußere Steckverbindungsöffnung (305) an einer Innenseite der äußeren Steckverbindungsdüse (310) gebildet ist, die Luftdüse des Kompressionsbandes (300) mit der äußeren Steckverbindungsdüse (310) ummantelt ist, und ein Elastizitätsmodul der Luftdüse des Kompressionsbandes (300) kleiner ist als ein Elastizitätsmodul des Hauptkörperelements (31).

11. Am Körper tragbare Vorrichtung (1000) nach einem der Ansprüche 1 bis 10, wobei die am Körper tragbare Vorrichtung (1000) ferner einen Luftsensor (50) umfasst und der Luftsensor (50) in dem Strömungsdurchgang (306) lokalisiert und an einer Innenwand des Strömungsdurchgangs (306) befestigt ist.

12. Am Körper tragbare Vorrichtung (1000) nach einem der Ansprüche 1 bis 11, wobei zwei äußere Steckverbindungsöffnungen (305), zwei Strömungsdurchgänge (306) und zwei innere Steckverbindungsöffnungen (307) vorhanden sind, die beiden äußeren Steckverbindungsöffnungen (305) eine erste äußere Steckverbindungsöffnung (305) und eine zweite äußere Steckverbindungsöffnung (305) sind, die beiden Strömungsdurchgänge (306) ein erster Strömungsdurchgang (306) und ein zweiter Strömungsdurchgang (306) sind, die beiden inneren Steckverbindungsöffnungen (307) eine erste innere Steckverbindungsöffnung (307) und eine zweite innere Steckverbindungsöffnung (307) sind, die erste äußere Steckverbindungsöffnung (305), der erste Strömungsdurchgang (306) und die erste innere Steckverbindungsöffnung (307) nacheinander in gegenseitiger Verbindung stehen, und die zweite äußere Steckverbindungsöffnung (305), der zweite Strömungsdurchgang (306) und die zweite innere Steckverbindungsöffnung (307) nacheinander in gegenseitiger Verbindung stehen;
die Blutdruckmessbaugruppe (40) einen Drucksensor und eine Luftpumpe umfasst, wobei eine Luftdüse des Drucksensors mit der ersten inneren Steckverbindungsöffnung (307) in gegenseitiger Verbindung steht und eine Luftdüse der Luftpumpe mit der zweiten inneren Steckverbindungsöffnung (307) in gegenseitiger Verbindung steht; und
das Kompressionsband (300) zwei Luftdüsen umfasst, wobei eine Luftdüse mit der ersten äußeren Steckverbindungsöffnung (305) in gegenseitiger Verbindung steht und die andere Luftdüse mit der zweiten äußeren Steckverbindungsöffnung (305) in gegenseitiger Verbindung steht.

13. Am Körper tragbare Vorrichtung (1000) nach Anspruch 12, wobei die Lünette (10) oder die untere Abdeckung (30) mit einem Entlüftungsloch (109) bereitgestellt ist und das Entlüftungsloch (109) mit dem inneren Hohlraum (110) des Ziffernblatts und einer Außenseite des Ziffernblatts (100) in gegenseitiger Verbindung steht.

14. Am Körper tragbare Vorrichtung (1000) nach einem der Ansprüche 1 bis 13, wobei die am Körper tragbare Vorrichtung (1000) ferner einen Herzfrequenzerfassungssensor umfasst, ein mittlerer Abschnitt der unteren Abdeckung (30) mit einem Montageloch bereitgestellt ist, das von der inneren Steckverbindungsöffnung (307) beabstandet ist, und der Herzfrequenzerfassungssensor in dem inneren Hohlraum (110) des Ziffernblatts untergebracht ist und dem Montageloch direkt gegenüberliegt oder zumindest teilweise in dem Montageloch untergebracht ist.

## Revendications

1. Dispositif pouvant être porté (1000), comprenant un cadran de montre (100), un bracelet de montre (200), et une bande de compression (300), dans lequel le cadran de montre (100) comprend une lunette (10), un couvercle inférieur (30), et un ensemble de mesure de la pression artérielle (40), la lunette (10) est reliée à un bord périphérique du couvercle inférieur (30) et entoure une cavité interne (110) du cadran de montre conjointement avec le couvercle inférieur (30),
dans lequel le couvercle inférieur (30) est pourvu d'un port de connexion par enfichage externe (305), d'un passage d'écoulement (306), et d'un port de connexion par enfichage interne (307) qui communiquent successivement, le port de connexion par enfichage externe (305) est plus proche de la lunette (10) que le port de connexion par enfichage interne (307), l'ensemble de mesure de la pression artérielle (40) est logé dans la cavité interne du cadran de montre (110) , et une buse d'air de l'ensemble de mesure de la pression artérielle (40) est en communication mutuelle avec le port de connexion par enfichage interne (307) ; et
le bracelet de montre (200) est connecté à la lunette (10), la bande de compression (300) et le bracelet de montre (200) sont empilés, et une buse d'air de la bande de compression (300) est en communication mutuelle avec le port de connexion par enfichage externe (305) ;
dans lequel le port de connexion par enfichage externe (305), le passage d'écoulement (306), et le port de connexion par enfichage interne (307) sont tous intégrés dans le couvercle inférieur (30) du cadran de montre (100).

2. Dispositif pouvant être porté (1000) selon la revendication 1, dans lequel le couvercle inférieur (30) comprend une face supérieure (301) qui fait face à la cavité interne (110) du cadran de montre, une distance entre le port de connexion par enfichage externe (305) et la face supérieure (301) du couvercle inférieur (30) forme une première plage de distance, une distance entre le port de connexion par enfichage interne (307) et la face supérieure (301) du couvercle inférieur (30) forme une seconde plage de distance, et la seconde plage de distance chevauche partiellement ou totalement la première plage de distance.

3. Dispositif pouvant être porté (1000) selon la revendication 1 ou 2, dans lequel le passage d'écoulement (306) est en forme de trait d'union, en forme de S, en forme de L, ou en forme de Z.

4. Dispositif pouvant être porté (1000) selon l'une quelconque des revendications 1 à 3, dans lequel le couvercle inférieur (30) comprend un élément de corps principal (31) et un premier élément de recouvrement (32), un bord périphérique de l'élément de corps principal (31) est relié à la lunette (10), le premier élément de recouvrement (32) est fixé à un côté de l'élément de corps principal (31), et le premier élément de recouvrement (32) forme le port de connexion par enfichage interne (307) et forme le passage d'écoulement (306) à travers l'enceinte conjointement avec l'élément de corps principal (31).

5. Dispositif pouvant être porté (1000) selon la revendication 4, dans lequel l'élément de corps principal (31) comprend une face supérieure (301) qui fait face à la cavité interne (110) du cadran de montre, la face supérieure (301) de l'élément de corps principal (31) fait partiellement saillie pour former un bossage, et le premier élément de recouvrement (32) est relié de manière fixe au bossage et forme le passage d'écoulement (306) à travers l'enceinte conjointement avec le bossage.

6. Dispositif pouvant être porté (1000) selon la revendication 4 ou 5, dans lequel la buse d'air de l'ensemble de mesure de la pression artérielle (40) est connectée par enfichage au port de connexion par enfichage interne (307), et un module d'élasticité de la buse d'air de l'ensemble de mesure de la pression artérielle (40) est supérieur à un module d'élasticité du premier élément de recouvrement (32).

7. Dispositif pouvant être porté (1000) selon l'une quelconque des revendications 4 à 6, dans lequel le couvercle inférieur (30) comprend également un second élément de recouvrement (34), le second élément de recouvrement (34) est fixé à un côté de l'élément de corps principal (31) qui est éloigné du premier élément de recouvrement (32), le second élément de recouvrement (34) forme le port de connexion par enfichage externe (305), la buse d'air de la bande de compression (300) est connectée par enfichage au port de connexion par enfichage externe (305), et un module d'élasticité de la buse d'air de la bande de compression (300) est supérieur à un module d'élasticité du second élément de recouvrement (34).

8. Dispositif pouvant être porté (1000) selon l'une quelconque des revendications 4 à 6, dans lequel l'élément de corps principal (31) comprend une face inférieure qui est éloignée de la cavité interne (110) du cadran de montre, la face inférieure de l'élément de corps principal (31) est partiellement en retrait pour former une rainure d'assemblage (319), une paroi de rainure de la rainure d'assemblage (319) fait partiellement saillie pour former une buse de connexion par enfichage externe (310), le port de connexion par enfichage externe (305) est formé sur un côté interne de la buse de connexion par enfichage externe (310), la buse d'air de la bande de compression (300) est située dans la rainure d'assemblage (319), la buse de connexion par enfichage externe (310) est connectée par enfichage à la buse d'air de la bande de compression (300), et un module d'élasticité de l'élément de corps principal (31) est supérieur à un module d'élasticité de la buse d'air de la bande de compression (300).

9. Dispositif pouvant être porté (1000) selon la revendication 8, dans lequel la buse de connexion par enfichage externe (310) est située entre la face supérieure (301) de l'élément de corps principal (31) et la face inférieure de l'élément de corps principal (31).

10. Dispositif pouvant être porté (1000) selon l'une quelconque des revendications 4 à 6, dans lequel une face périphérique de l'élément de corps principal (31) fait partiellement saillie pour former une buse de connexion par enfichage externe (310), le port de connexion par enfichage externe (305) est formé sur un côté interne de la buse de connexion par enfichage externe (310), la buse d'air de la bande de compression (300) est emmanchée avec la buse de connexion par enfichage externe (310), et un module d'élasticité de la buse d'air de la bande de compression (300) est inférieur à un module d'élasticité de l'élément de corps principal (31).

11. Dispositif pouvant être porté (1000) selon l'une quelconque des revendications 1 à 10, dans lequel le dispositif pouvant être porté (1000) comprend également un capteur d'air (50), et le capteur d'air (50) est situé dans le passage d'écoulement (306) et fixé à une paroi interne du passage d'écoulement (306).

12. Dispositif pouvant être porté (1000) selon l'une quelconque des revendications 1 à 11, dans lequel il existe deux ports de connexion par enfichage externes (305), deux passages d'écoulement (306), et deux ports de connexion par enfichage internes (307), les deux ports de connexion par enfichage externes (305) sont un premier port de connexion par enfichage externe (305) et un second port de connexion par enfichage externe (305), les deux passages d'écoulement (306) sont un premier passage d'écoulement (306) et un second passage d'écoulement (306), les deux ports de connexion par enfichage internes (307) sont un premier port de connexion par enfichage interne (307) et un second port de connexion par enfichage interne (307), le premier port de connexion par enfichage externe (305), le premier passage d'écoulement (306), et le premier port de connexion par enfichage interne (307) communiquent successivement, et le second port de connexion par enfichage externe (305), le second passage d'écoulement (306), et le second port de connexion par enfichage interne (307) communiquent successivement ;
l'ensemble de mesure de la pression artérielle (40) comprend un capteur de pression et une pompe à air, une buse d'air du capteur de pression est en communication mutuelle avec le premier port de connexion par enfichage interne (307), et une buse d'air de la pompe à air est en communication mutuelle avec le second port de connexion par enfichage interne (307) ; et
la bande de compression (300) comprend deux buses d'air, une buse d'air communique avec le premier port de connexion par enfichage externe (305), et l'autre buse d'air communique avec le second port de connexion par enfichage externe (305).

13. Dispositif pouvant être porté (1000) selon la revendication 12, dans lequel la lunette (10) ou le couvercle inférieur (30) est pourvu d'un trou de respiration (109), et le trou de respiration (109) communique avec la cavité interne (110) du cadran de montre et un extérieur du cadran de montre (100).

14. Dispositif pouvant être porté (1000) selon l'une quelconque des revendications 1 à 13, dans lequel le dispositif pouvant être porté (1000) comprend également un capteur de détection de fréquence cardiaque, une partie médiane du couvercle inférieur (30) est pourvue d'un trou de montage espacé du port de connexion par enfichage interne (307), et le capteur de détection de fréquence cardiaque est logé dans la cavité interne (110) du cadran de la montre, et fait directement face au trou de montage ou est au moins partiellement logé dans le trou de montage.
